# EUROPEAN PATENT APPLICATION

(11) **EP 2 316 493 A2**
(43) Date of publication of application: **04.05.2011**
(21) Application number: 11155333.5
(22) Date of filing: 07.11.2003
(51) Int. Cl.: A61K 51/04, A61K 51/10

(54) **Ethylenedicysteine (EC)-drug conjugates, compositions and methods for tissue specific disease imaging**

(30) Priority: 07.11.2002 US 424493 P
(62) Divisional of application: 03811262.9
(71) Applicant: Board of Regents, The University of Texas System, Austin, TX 78701 (US); Cellpoint LLC, Englewood, CO 80111 (US)
(72) Inventor: Yang, David, J, Houston, TX 77030 (US); Yu, Dong-fang, Houston, TX 77030 (US); Oh, Chang-Sok, Houston, TX 77077 (US); Bryant, Jerry, L., Jr, Houston, TX 77030 (US)
(74) Representative: Dehmel, Albrecht

(57) **Abstract**

The present invention relates to a radionuclide-labeled ethylenedicysteine (EC) derivative comprising a disease cell cycle targeting compound, wherein
(i) the EC forms an N₂S₂ chelate with the radionuclide,
(ii) the disease cell cycle targeting compound is a nucleoside, or analog thereof, and is selected from guanine, adenosine, penciclovir, FIAU, FIRU, FMAU, IVFRU, GCV, PCV, FGCV, FPCV, FHPG, and FHBG; and
(iii)the disease cell cycle targeting compound is conjugated to EC directly or via a linker.

## Description

### I. Field of the Invention

The present invention relates generally to the fields of labeling, radioimaging, radioimmunotherapy, and chemical synthesis. More particularly, it concerns a strategy for radiolabeling target ligands. It further concerns methods of using those radiolabeled ligands to target tumor angiogenesis, hypoxia, apoptosis, disease receptors, disease functional pathways, and disease cell cycles, as well as for the assessment of pharmaceutical agent effectiveness on these biochemical processes.

### II. Description of Related Art

Angiogenesis, the proliferation of endothelial and smooth muscle cells to form new blood vessels, is an essential component of the metastatic pathway. These vessels provide the principal route by which certain cells exit the primary tissue site and enter the circulation. For many disease tissue, the vascular density can provide a prognostic indicator of metastatic potential or survival, with highly vascularized tumors having a higher incidence of metastasis than poorly vascularized tissues (Bertolini *et al.,* 1999; Cao, 1999; Smith *et al.,* 2000).

It may be feasible to block angiogenesis and tumor progression by using anti-angiogenic agents. At present, antiangiogenic agents under clinical testing include: naturally occurring inhibitors of angiogenesis (e.g. angiostatin, endostatin, platelet factor-4), (Jiang *et al.,* 2001; Dhanabal *et al.*, 1999; Moulton *et al.*, 1999; Jouan *et al.*, 1999) specific inhibitors of endothelial cell growth (e.g. TNP-470, thalidomide, interleukin-12), (Logothetis *et al.*, 2001; Moreira *et al*., 1999; Duda *et al*., 2000) agents neutralizing angiogenic peptides (e.g. antibodies to fibroblast growth factor or vascular endothelial growth factor, suramin and analogues, tecogalan) (Bocci *et al.*, 1999; Sakamoto *et al*., 1995) or their receptors, (Pedram *et al*., 2001) agents that interfere with vascular basement membrane and extracellular matrix (e.g. metalloprotease inhibitors, angiostatic steroids), (Lozonschi *et al.*, 1999; Maekawa *et al*., 1999; Banerjeei *et al.*, 2000) anti-adhesion molecules, (Liao *et al*., 2000) antibodies such as anti-integrin αᵥβ₃, (Yeh *et al.*, 2001) and miscellaneous drugs that modulate angiogenesis by diverse mechanisms of action (Gasparini 1999).

For example many malignant tumors are angiogenesis-dependent. Several experimental studies suggest that primary tumor growth, invasiveness and metastasis require neovascularization (Sion-Vardy *et al.*, 2001; Guang-Wu *et al*., 2000; Xiangming *et al*., 1998). Tumor-associated angiogenesis is a complex, multi-step process under the control of positive and negative soluble factors. Acquisition of the angiogenic phenotype is a common pathway for tumor progression, and active angiogenesis is associated with molecular mechanisms leading to tumor progression (Ugurel *et al*., 2001). For instance, vascular endothelial growth factor (VEGF) is a mitogen, morphogen and chemoattractant for endothelial cells and, *in vivo,* is a powerful mediator of vessel permeability (Szus *et al*., 2000). Interleukin-8 (IL-8) is a chemoattractant for neutrophils and is a potent angiogenic factor (Petzelbauer *et al.*, 1995). Basic fibroblast growth factor (bFGF) has been associated with tumorigenesis and metastasis in several human cancers (Smith *et al.*, 1999). The prognostic value of angiogenesis factor expression (e.g. VEGF, bFGF, microvessel density, IL-8, MMP-2 and MMP-9) has been determined for cancer patients treated with chemotherapy (Inoue *et al.*, 2000; Burian *et al.*, 1999). These factors regulate metastasis and angiogenesis and may predict the metastatic potential in individual cancer patients (Slaton *et al.*, 2001).

Apoptosis defects in programmed cell death play an important role in tumor pathogenesis. These defects allow neoplastic cells to survive beyond their normal intended lifespan, and subvert the need for exogenous survival factors. Apoptosis defects also provide protection from hypoxia and oxidative stress as the tumor mass expands. They also allow time for genetic alterations that deregulate cell proliferation to accumulate, resulting in interference with differentiation, angiogenesis, and increased cell motility and invasiveness during tumor progression (Reed, 1999). In fact, apoptosis defects are recognized as an important complement to protooncogene activation, as many deregulated oncoproteins that drive cell division also trigger apoptosis (Green and Evan, 2002). Similarly, defects in DNA repair and chromosome segregation normally trigger cell suicide as a defense mechanism for readicating genetically unstable cells. Thus, apoptosis defects permit survival of genetically unstable cells, providing opportunities for selection of progressively aggressive clones (Ionov *et al.*, 2000). Apoptosis defects also facilitate metastasis by allowing epithelial cells to survive in a suspended state, without attachment to extracellular matrix (Frisch and Screaton, 2001). They also promote resistance to the immune system, inasmuch as many of the weapons used for attacking tumors, including cytolytic T cells (CTLs) and natural killer (NK) cells, depend on the integrity of the apoptosis machinery (Tschopp *et al.*, 1999). Finally, cancer-associated defects in apoptosis play a role in chemoresistance and radioresistance, increasing the threshold for cell death and thereby requiring higher doses for tumor killing (Makin and Hickman, 2000). Thus, defective apoptosis regulation is a fundamental aspect of the biology of cancer.

When it comes to the successful eradication of cancer cells by nonsurgical means, all roads ultimately lead to angiogenesis and apoptosis. Essentially all cytotoxic anticancer drugs currently in clinical use block angiogenesis and induce apoptosis of malignant cells. While microtubule binding drugs, DNA-damaging agents, and nucleosides are important weapons in the treatment of cancer, new classes of targeted therapeutics may soon be forthcoming. These new classes of targeted therapeutics may soon be forthcoming based on strategies that have emerged from a deeper understanding of the molecular mechanisms that underlie the phenomenon of angiogenesis and apoptosis (Reed, 2003).

Though angiogenic and apoptotic factors reflect angiogenesis and apoptosis status, these agents may not adequately reflect the therapeutic response of tumors. Currently, methods of assessing angiogenesis and apoptosis in tumors rely on counting microvessel density in the areas of neovascularization and observing annexin V with FACS techniques. After tissue biopsy, immunohistochemistry of tissue specimen is then performed. Both techniques are invasive and cannot be repeatedly performed.

Improvement of scintigraphic tumor imaging is extensively determined by development of more tumor specific radiopharmaceuticals. Due to greater tumor specificity, radiolabeled ligands as well as radiolabeled antibodies have opened a new era in scintigraphic detection of tumors and undergone extensive preclinical development and evaluation (Mathias *et al.*, 1996, 1997a, 1997b). Radionuclide imaging modalities (positron emission tomography, PET; single photon emission computed tomography, SPECT) are diagnostic cross-sectional imaging techniques that map the location and concentration of radionuclide-labeled radiotracers. Although CT and MRI provide considerable anatomic information about the location and the extent of tumors, these imaging modalities cannot adequately differentiate invasive lesions from edema, radiation necrosis, grading or gliosis. PET and SPECT can be used to localize and characterize tumors by measuring metabolic activity.

[¹⁸F]FMISO has been used to diagnose head and neck tumors, myocardial infarction, inflammation, and brain ischemia (Martin *et al.* 1992; Yeh *et al.* 1994; Yeh *et al.* 1996; Liu *et al.* 1994). Tumor to normal tissue uptake ratio was used as a baseline to assess tumor hypoxia (Yet *et al.* 1996). Although tumor metabolic imaging using [¹⁸F]FDG was clearly demonstrated, introducing molecular imaging agents into clinical practice depends on some other factors such as easy availability and isotope cost. [¹⁸F]fluorodeoxyglucose (FDG) has been used to diagnose tumors, myocardial infarction, and neurological disease. In addition, PET radiosynthesis must be rapid because of short half-life of the positron isotopes. ¹⁸F chemistry is complex and is not reproducible in different molecules.

Several compounds have been labeled with ^{99m}Tc using nitrogen and sulfur chelates (Blondeau *et al.*, 1967; Davison *et al.*, 1980). Bis-aminoethanethiol tetradentate ligands, also called diaminodithol compounds, are known to form very stable Tc(V)O complexes on the basis of efficient binding of the oxotechnetium group to two thiolsulfur and two amine nitrogen atoms. Radiometal complexes of 2-pyrrolthiones labeled with ^{99m}Tc-2-pyrrolthiones complexes have been developed for use as radiopharmaceuticals for imaging and therapy (WO 0180906A2). ^{99m}Tc-L,L-ethylenedicysteine (^{99m}Tc-EC) is a recent and successful example of N₂S₂ chelates. EC can be labeled with ^{99m}Tc easily and efficiently with high radiochemical purity and stability, and is excreted through the kidney by active tubular transport (Surma *et al.*, 1994; Van Nerom *et al.*, 1990, 1993; Verbruggen *et al.,* 1990, 1992). Furthermore, ^{99m}Tc chelated with ethylenedicysteine (EC) and conjugated with a variety of ligands has been developed for use as an imaging agent for tissue-specific diseases, a prognostic tool or as a tool to deliver therapeutics to specific sites within a mammalian body (WO 0191807A2, AU 0175210A5). ^{99m}Tc-EC-chelates have been developed for renal imaging and examination of renal function (US 5986074 and US 5955053). A method of preparing ^{99m}Tc-EC complexes and a kit for performing said method has also been developed (US 5268163 and WO 9116076A1).

However, there still exist a need for the development of new agents to target tumor angiogenesis, hypoxia, apoptosis defects, disease receptors, disease functional pathways, and disease cell cycles, as well as for the assessment of the pharmaceutical agent effectiveness on these biochemical processes.

### SUMMARY OF THE INVENTION

Certain embodiments of the present invention are generally concerned with compounds that comprises an N₂S₂ chelate conjugated to a targeting ligand, wherein the targeting ligand is a disease cell cycle targeting compound, a tumor angiogenesis targeting ligand, a tumor apoptosis targeting ligand, a disease receptor targeting ligand, amifostine, angiostatin, monoclonal antibody C225, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine.

A particular aspect of the invention comprises an N₂S₂ chelate compound of the formula: 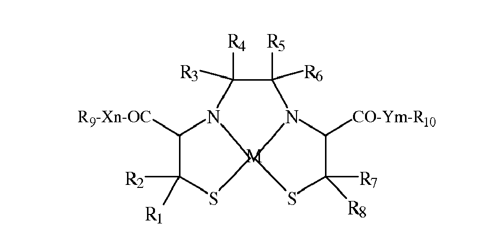 wherein R₁, R₂, R₃, R₄, R₅, R₆, R₇ and R₈ are independently H or CH₃; R₉ is H, CH₃, a disease cell cycle targeting compound, amifostine, angiostatin, anti-EGF receptor receptor, monoclonal antibody CD31, monoclonal antibody CD40, capecitabine, a COX-2, deoxycytidine, fullerene, herceptin, human serum albumin, lactose, leuteinizing hormone, pyridoxal, quinazoline, thalidomide, transferrin, or trimethyl lysine; R₁₀ is H, CH₃, disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; n is 0 or 1; m is 0 or 1; X is a water soluble peptide, C₁-C₂₀ alkyl, glutamic acid, polyglutamic acid, aspartic acid, polyaspartic acid, bromoethylacetate, ethylenediamine or lysine when n is 1, or a bond when n is 0; Y is a water soluble peptide, C₁-C₂₀ alkyl, glutamic acid, polyglutamic acid, aspartic acid, polyaspartic acid, bromoethylacetate, ethylenediamine or lysine when m is 1, or a bond when m is 0; and M is ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu.

In a preferred embodiment the compound as previously described wherein R₉ and R₁₀ are independently H, CH₃, COX-2, anti-EGF receptor, herceptin, angiostatin, or thalidomide; and M is *^{99m}*Tc.

In another preferred embodiment the compound as previously described wherein said disease cell cycle targeting compound is adenosine or penciclovir.

Angiogenesis targeting refers to the use of an agent to bind to neovascularization, such as neovascularization of tumor cells. This is discussed in greater detail in the specification below. Agents that are used for this purpose are known to those of ordinary skill in the art for use in performing various tumor measurements, including measurement of the size of a tumor vascular bed, and measurement of tumor volume. Some of these agents bind to the vacular wall. One of ordinary skill in the art would be familiar with the agents that are available for use for this purpose.

Tumor apoptosis targeting refers to use of an agent to bind to a cell that is undergoing apoptosis or at risk of undergoing apoptosis. These agents are generally used to provide an indicator of the extent or risk of apoptosis, or programmed cell death, in a population of cells, such as a tumor. One of ordinary skill in the art would be familiar with agents that are used for this purpose. Tumor apoptosis targeting is discussed in greater detail in the specification below.

In disease receptor targeting, certain ligands are exploited for their ability to bind to particular cellular receptors that are overexpressed in disease states, such as cancer. Examples of such receptors which are targeted include estrogen receptors, androgen receptors, pituitary receptors, transferrin receptors, and progesterone receptors. Examples of agents that can be applied in disease-receptor targeting are shown in Table 1. Disease receptor targeting is discussed in greater detail in the specification below.

Disease cell cycle targeting refers to targeting of agents that are upregulated in proliferating cells. Compounds used for this purpose can be used to measure various parameters in cells, such as tumor cell DNA content. Many of these agents are nucleoside analogues. Further discussion pertaining to disease cell cycle targeting is provided in the specification below.

Certain drug-based ligands of the present invention can be applied in measuring the pharmacological response of a subject to a drug. A wide range of parameters can be measured in determining the response of a subject to administration of a drug. One of ordinary skill in the art would be familiar with the types of responses that can be measured. These responses depend in part upon various factors, including the particular drug that is being evaluated, the particular disease or condition for which the subject is being treated, and characteristics of the subject. Radiolabeled agents can be applied in measuring drug assessment. Further discussion pertaining to drug assessment is provided in other parts of this specification.

Another aspect of the current invention comprises a method of synthesizing a radiolabeled ethylenedicysteine derivative for imaging comprising the steps: a) obtaining a compound amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, trimethyl lysine, or a disease cell cycle targeting compound; b) admixing said compound with ethylenedicysteine (EC) to obtain an EC-compound derivative; and c) admixing said EC-compound derivative with a radionuclide and a reducing agent to obtain a radionuclide labeled EC-compound derivative, wherein the EC forms an N₂S₂ chelate with the radionuclide.

In one embodiment the reducing agent may be a dithionite ion, a stannous ion or a ferrous ion. The radionuclide is preferably *^{99m}*Tc.

In yet another embodiment, the disease cell cycle targeting compound is adenosine or penciclovir.

Another aspect of the current invention comprises a method of imaging a site within a mammalian body comprising the steps: a) administering an effective diagnostic amount of an N₂S₂ chelate compound of the formula: 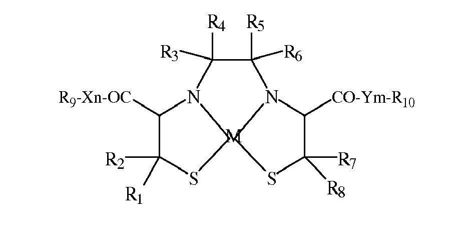 wherein R₁, R₂, R₃, R₄, R₅, R₆, R₇ and R₈ are independently H or CH₃; R₉ is H, CH₃, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; R₁₀ is H, CH₃, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; n is 0 or 1; m is 0 or 1; X is a water soluble peptide, C₁-C₂₀ alkyl, glutamic acid, polyglutamic acid, aspartic acid, polyaspartic acid, bromoethylacetate, ethylenediamine or lysine when n is 1, or a bond when n is 0; Y is a water soluble peptide, C₁-C₂₀ alkyl, glutamic acid, polyglutamic acid, aspartic acid, polyaspartic acid, bromoethylacetate, ethylenediamine or lysine when m is 1, or a bond when m is 0; M is *^{99m}*T_{c}, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu; and b) detecting a radioactive signal from said compound localized at a site. Preferably M is *^{99m}*Tc. The disease cell cycle targeting compound may be adenosine or penciclovir. The site may be a tumor, an infection, breast cancer, ovarian cancer, prostate cancer, endometrium, heart cancer, lung cancer, brain cancer, liver cancer, folate (+) cancer, ER (+) cancer, spleen cancer, pancreas cancer, or intestine cancer.

Yet another aspect of the invention comprises a kit for preparing a radiopharmaceutical preparation comprising: a) a sealed container including a predetermined quantity of a compound of the formula: 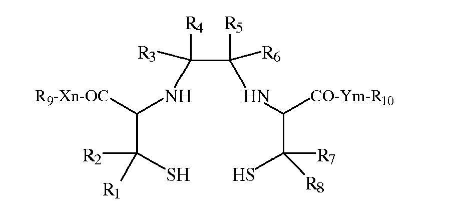 wherein R1, R2 , R3, R4 , R5 , R6 , R7 and R8 are independently H or CH3; R9 is H, CH3, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; R10 is H, CH3, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; n is 0 or 1; m is 0 or 1; X is a water soluble peptide, C1-C20 alkyl, glutamic acid, polyglutamic acid, aspartic acid, polyaspartic acid, bromoethylacetate, ethylenediamine or lysine when n is 1, or a bond when n is 0; Y is a water soluble peptide, C1-C20 alkyl, glutamic acid, polyglutamic acid, aspartic acid, polyaspartic acid, bromoethylacetate, ethylenediamine or lysine when m is 1, or a bond when m is 0; and b) a sufficient amount of a reducing label conjugated with *^{99m}*Tc, *¹⁸⁸*Re, *¹⁸⁶*Re, *¹⁸³*Sm, *¹⁶⁶*Ho, *⁹⁰*Y, *⁸⁹*Sr, *⁶⁷*Ga, *⁶⁸*Ga, *¹¹¹*In, ¹⁸³Gd, *⁵⁹*Fe, *²²⁵*Ac, *²¹²*Bi, *²¹¹*At, ⁴⁵Ti, ⁶⁰Cu, ⁶¹Cu, ⁶⁷Cu, *⁶⁴*Cu or *⁶²*Cu. The reducing label may be conjugated with *^{99m}Tc.* The disease cell cycle targeting compound may be adenosine or penciclovir.

An aspect of the current invention comprises a reagent for preparing a scintigraphic imaging agent comprising an N₂S chelate compound of the formula: 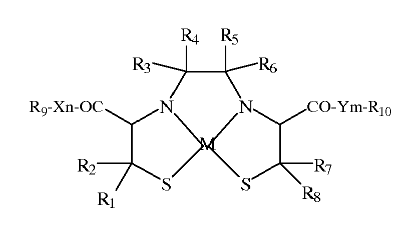 wherein R₁, R₂, R₃, R₄ , R₅, R₆, R₇ and R₈ are independently H or CH₃; R₉ is H, CH₃, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; R₁₀ is H, CH₃, a disease cell cycle targeting compound, amifostine, angiostatin, anti-EGF receptor, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine; n is 0 or 1; m is 0 or 1; X is a water soluble peptide, C₁-C₂₀ alkyl, glutamic acid, polyglutamic acid, aspartic acid, polyaspartic acid, bromoethylacetate, ethylenediamine or lysine when n is 1, or a bond when n is 0; Y is a water soluble peptide, C₁-C₂₀ alkyl, glutamic acid, polyglutamic acid, aspartic acid, polyaspartic acid, bromoethylacetate, ethylenediamine or lysine when m is 1, or a bond when m is 0; and M is *^{99m}*Tc, ¹⁸⁸Re, ¹⁸⁶Re, ¹⁸³Sm, ¹⁶⁶Ho, ^{9o}Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu. M is preferably *^{99m}*Tc and the disease cell cycle targeting compound is preferably adenosine or penciclovir.

The present invention also pertains to methods of assessing the pharmacology of an agent of interest, comprising (1) preparing a conjugate of the agent to an N₂S₂ chelate, (2) adding a radioactive nuclide to said conjugated chelate to form a radioactive conjugate; (3) administering said radioactive conjugate to a subject; and (4) assessing the pharmacology of the agent. The agent of interest may be a pharmaceutical agent. The N₂S₂ chelate in certain embodiments is ethylenedicysteine. The subject may be any subject, such as a laboratory animal or a human. In certain embodiments, assessing the pharmacology of the agent comprises assessing the biodistribution of the agent, assessing the biostability of the agent, or assessing the bioelimination of the agent.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1** *In vitro* cellular uptake of *^{99m}*Tc-EC-Angiostatin in breast cancer cells
**FIG. 2** *In vitro* blocking study of *^{99m}*Tc-EC-Angiostatin in breast cancer cells with
   unlabeled angiostatin
**FIG. 3** Percent inhibition of *^{99m}*Tc-EC-Angiostatin after adding unlabeled angiostatin
**FIG. 4** Tumor uptake of *^{99m}*Tc-EC-anti-angiogenic agents in breast tumor-bearing rats
**FIG. 5** Tumor-to-muscle count density ratios of *^{99m}*Tc-EC-anti-angiogenic agents
**FIG. 6** Scintigraphic images of *^{99m}*Tc-EC-angiostatin
**FIG. 7** HPLC chromatogram of *^{99m}*Tc-EC-C225
**FIG. 8** *In vitro* comparison of EC-C225 and C225
**FIG. 9** Biodistribution of *^{99m}*Tc-EC-C225 in A431 vulvic tumor-bearing nude mice
**FIG. 10** Tumor-to-tissue count density ratios of *^{99m}*Tc-EC-C225 in A431 vulvic tumor-bearing nude mice
**FIG.11** Case study 1: *^{99m}*Tc-EC-C225 scan for head and neck cancer: left
   jugulodigastric lymph node
**FIG. 12** Case study 2: *^{99m}*Tc-EC-C225 scan for head and neck cancer: left base of
   tongue and floor of mouth
**FIG. 13** Synthesis of *^{99m}*Tc-EC-COX-2
**FIG. 14** NMR spectra data of EC-COX-2-ester
**FIG. 15** NMR spectra data of EC-COX-2
**FIG. 16** *In vitro* cellular uptake of *^{99m}*Tc-EC-agents in breast cancer cells
**FIG. 17** Scintigraphic Images of *^{99m}*Tc-EC-COX-2
**FIG. 18** Chemical structure of EC-thalidomide
**FIG. 19** Chemical structure of EC-quinazoline
**FIG. 20** Synthesis of aminopenciclovir
**FIG. 21** Synthesis of EC-aminopenciclovir (EC-Guanine)
**FIG. 22** *In vitro* cellular uptake of *^{99m}*Tc-EC-aminopenciclovir (99mTc-EC-Guanine)
   in human cancer cell lines
**FIG. 23** Scintigraphic images of *^{99m}*Tc-EC-aminopenciclovir (*^{99m}*Tc-EC-Guanine)
**FIG. 24** Autoradiogram of *^{99m}*Tc-EC-aminopenciclovir (*^{99m}*Tc-EC-Guanine)
**FIG. 25** Chemical structure of EC-deoxycytidine
**FIG. 26** Chemical structure of capecitabine
**FIG. 27** Synthesis of EC-adenosine
**FIG. 28** Autoradiogram of 99mTc-EC-adenosine
**FIG. 29** Scintigraphic images of *^{99m}*Tc-EC-LHRH
**FIG. 30** *In vitro* cellular uptake of *^{99m}*Tc-EC-agents in human ovarian cancer cells
**FIG. 31** Scintigraphic images of *^{99m}*Tc-EC-LH
**FIG. 32** Autoradiogram of *^{99m}*Tc-EC-Transferrin
**FIG. 33** Synthesis of EC-TML
**FIG. 34** Mass spectrum of EC-TML
**FIG. 35** Biodistribution of *^{99m}*Tc-EC-TML in breast tumor-bearing rats
**FIG. 36** Scintigraphic Images of *^{99m}*Tc-EC-TML
**FIG. 37** Synthesis of EC-pyridoxal
**FIG. 38** Synthesis of *^{99m}*Tc-fullerene-EC-drug conjugates
**FIG. 39** Synthesis of ¹⁸⁸Re-EC deoxyglucose
**FIG. 40** Synthesis of ¹⁸⁸Re-EC-metronidazole
**FIG. 41** *In vitro* cell culture of ¹⁸⁸Re- and *^{99m}*Tc-labeled EC-penciclovir (EC-Guanine).
**FIG. 42** *In vitro* cell culture of ¹⁸⁸Re-EC-metronidazole
**FIG. 43A-43C** *In vitro* cell culture of *^{99m}*Tc-EC-deoxyglucose (kit formulation)

### I. The Present Invention

The present invention overcomes deficiencies in the art by providing novel radiolabled-EC conjugates designed to target tumor angiogenesis, hypoxia, apoptosis defects, disease receptors, disease function pathways, and disease cell cycles. The radiolabled-EC conjugates can also be used to assess the effectiveness of pharmaceutical agents on the biochemical processes stated above. More particularly, the present invention provides the radiolabled *^{99m}*Tc-EC conjugates to target tumor angiogenesis, hypoxia, apoptosis defects, disease receptors, disease function pathways, and disease cell cycles, as well as for the assessment of a pharmaceutical agent effectiveness on these biochemical processes.

### II. Radiopharmaceuticals

In the field of nuclear medicine, certain pathological conditions are localized, or their extent is assessed, by detecting the distribution of small quantities of internally-administered radioactively labeled tracer compounds (called radiotracers or radiopharmaceuticals). Methods for detecting these radiopharmaceuticals are known generally as imaging or radioimaging methods.

### III. Radioimaging Methods

In radioimaging, the radiolabel is a gamma-radiation emitting radionuclide and the radiotracer is located using a gamma-radiation detecting camera (this process is often referred to as gamma scintigraphy). The imaged site is detectable because the radiotracer is chosen either to localize at a pathological site (termed positive contrast) or, alternatively, the radiotracer is chosen specifically not to localize at such pathological sites (termed negative contrast).

### IV. Radionuclides

A variety of radionuclides are known to be useful for radioimaging and radioimmunotherapy, including ⁶⁷Ga/⁶⁸Ga, *^{99m}*Tc, ¹¹¹In, ¹²³I, ¹²⁵I, ¹⁶⁹Yb or ¹⁸⁶Re/¹⁸⁸Re. Due to better imaging characteristics and lower price, attempts have been made to replace or provide an alternative to ¹²³I, ¹³¹I, ⁶⁷Ga and ¹¹¹In labeled compounds with corresponding *^{99m}*Tc labeled compounds when possible. Due to favorable physical characteristics as well as extremely low price ($0.21/mCi), *^{99m}*Tc is preferred to label radiopharmaceuticals. Although it has been reported that DTPA-drug conjugate could be labeled with *^{99m}*Tc effectively (Mathias *et al.*, 1997), DTPA moiety does not chelate with *^{99m}*Tc as stable as with ¹¹¹In (Goldsmith, 1997).

A number of factors must be considered for optimal radioimaging in humans. To maximize the efficiency of detection, a radionuclide that emits gamma energy in the 100 to 200 keV range is preferred. To minimize the absorbed radiation dose to the patient, the physical half-life of the radionuclide should be as short as the imaging procedure will allow. To allow for examinations to be performed on any day and at any time of the day, it is advantageous to have a source of the radionuclide always available at the clinical site. *^{99m}*Tc is a preferred radionuclide because it emits gamma radiation at 140 keV, it has a physical half-life of 6 hours, and it is readily available on-site using a molybdenum-99/technetium-99m generator.

### V. Ethylenedicysteine

The present invention utilizes *^{99m}*Tc-EC conjugates as a labeling agent to target tumor angiogenesis, hypoxia, apoptosis defects, disease receptors, disease functional pathways, and disease cell cycles, as well as for the assessment of a pharmaceutical agent's effectiveness on these biochemical processes.

The advantage of conjugating the EC with tissue targeting ligands is that the specific binding properties of the tissue targeting ligand concentrates the radioactive signal over the area of interest. It is envisioned that the use of *^{99m}*Tc-EC as a labeling strategy can be effective with ligands designed for targeting disease receptors, hypoxia, apoptosis pathways, disease cell cycles, disease functional pathways, radioimmunotherapy, and assessment of a pharmaceutical agent's effectiveness on these biochemical processes. Examples of certain embodiments for the present invention can be found in Table 1.

**Table 1**

| **Targets for EC-Complex** | **Examples** |
|---|---|
| Tumor Angiogenesis | *^{99m}*Tc-EC-celebrex (EC-COX-2), *^{99m}*Tc-EC-C225, and *^{99m}*Tc-EC-angiostatin |
| Disease Receptor | *^{99m}*Tc-EC-leuteinizing hormone (*^{99m}*Tc-EC-LH antibody), *^{99m}*Tc-EC-transferrin, *^{99m}*Tc-EC-somatostatin, *^{99m}*Tc-EC-androgen, *^{99m}*Tc-EC-estrogen, *^{99m}*Tc-EC-progesterone |
| Disease Cell Cycle | *^{99m}*Tc-EC-adenosine, and *^{99m}*Tc-EC-penciclovir |
| Pharmaceutical Agent Assessment | *^{99m}*Tc-EC-carnitine, *^{99m}*Tc-EC-puromycin |
| Apoptosis Targeting | *^{99m}*Tc-EC-TRAIL, *^{99m}*Tc-EC-caspase-3 |
| | |

### VI. ^{99m}Technetium-EC Complex

*^{99m}Tc* is normally obtained as *^{99m}*Tc pertechnetate (TcO₄⁻; technetium in the +7 oxidation state), usually from a molybdenum-99/technetium-*99m* generator. However, pertechnetate does not bind well with other compounds. Therefore, in order to radiolabel a compound, *^{99m}*Tc pertechnetate must be converted to another form. Since technetium does not form a stable ion in aqueous solution, it must be held in such solutions in the form of a coordination complex that has sufficient kinetic and thermodynamic stability to prevent decomposition and resulting conversion of *^{99m}*Tc either to insoluble technetium dioxide or back to pertechnetate.

For the purpose of radiolabeling, it is particularly advantageous for the *^{99m}*Tc complex to be formed as a chelate in which all of the donor groups surrounding the technetium ion are provided by a single chelating ligand - in this case, ethylenedicysteine. This allows the chelated *^{99m}*Tc to be covalently bound to a tissue specific ligand either directly or through a single linker between the ethylenedicysteine and the ligand.

Technetium has a number of oxidation states: +1, +2, +4, +5, +6 and +7. When it is in the +1 oxidation state, it is called Tc MIBI. Tc MIBI must be produced with a heat reaction (Seabold *et al.* 1999). For purposes of the present invention when using the N₂S₂ chelate, it is important that the Tc be in the +4 oxidation state. This oxidation state is ideal for forming the N₂S₂ chelate with EC. Thus, in forming a complex of radioactive technetium with the drug conjugates of the invention, the technetium complex, preferably a salt of *^{99m}*Tc pertechnetate, is reacted with the drug conjugates of the invention in the presence of a reducing agent.

The preferred reducing agent for use in the present invention is stannous ion in the form of stannous chloride (SnCl₂) to reduce the Tc to its +4 oxidation state. However, it is contemplated that other reducing agents, such as dithionate ion or ferrous ion may be useful in conjunction with the present invention. It is also contemplated that the reducing agent may be a solid phase reducing agent. The amount of reducing agent can be important as it is necessary to avoid the formation of a colloid. It is preferable, for example, to use from about 10 to about 100µg SnCl₂ per about 100 to about 300 mCi of Tc pertechnetate. The most preferred amount is about 0.1 mg SnCl₂ per about 200 mCi of Tc pertechnetate and about 2 ml saline. This typically produces enough Tc-EC-tissue specific ligand conjugate for use in 5 patients.

It is often also important to include an antioxidant and a transition chelator in the composition to prevent oxidation of the ethylenedicysteine. The preferred antioxidant for use in conjunction with the present invention is vitamin C (ascorbic acid). However, it is contemplated that other antioxidants, such as tocopherol, pyridoxine, thiamine or rutin, may also be useful. Examples of transition chelators include glucoheptonate, gluconate, glucarate, citrate, and tartarate. In certain embodiments, the transition chelator is either gluconate or glucarate, neither of which interferes with the stability of ethylenedicysteine.

### VII. EC Ligands

In general, the ligands for use in conjunction with the present invention will possess either amino or hydroxy groups that are able to conjugate to EC on either one or both acid arms. If amino or hydroxy groups are not available (e.g., acid functional group), a desired ligand may still be conjugated to EC and labeled with *^{99m}*Tc using the methods of the invention by adding a linker, such as ethylenediamine, amino propanol, diethylenetriamine, aspartic acid, polyaspartic acid, glutamic acid, polyglutamic acid, or lysine. Ligands contemplated for use in the present invention include, but are not limited to, angiogenesis/antiangiogenesis ligands, DNA topoisomerase inhibitors, glycolysis markers, antimetabolite ligands, apoptosis/hypoxia ligands, DNA intercalators, cell receptor markers, peptides, nucleotides, antimicrobials such as antibiotics or antifungals, organ specific ligands and sugars or agents that mimic glucose.

EC itself is water soluble. It is necessary that the EC-drug conjugate of the invention also be water soluble. Many of the ligands used in conjunction with the present invention will be water soluble, or will form a water soluble compound when conjugated to EC. If the tissue specific ligand is not water soluble, however, a linker which will increase the solubility of the ligand may be used. Linkers may attach to an aliphatic, aromatic alcohol, amine, peptide, carboxylic, peptide or any combination thereof. Linkers may be either poly amino acid (peptide), an amino acid, alanine arginine, asparagine, aspartate, cysteine, glutamate, glutamine, glycine, proline, serine, tyrosine, histidine, isoleucine, leucine, lysine, methionine, phenylalanine, threonine, tryptophan, valine or any combination thereof. More preferably linkers may be glutamic acid, aspartic acid, lysine or any combination thereof.

It is also envisioned that the EC-tissue specific ligand drug conjugates of the current invention may be chelated to other radionuclides and used for radionuclide therapy. Generally, it is believed that virtually any α, β-emitters, γ-emitter, or β, γ-emitter can be used in conjunction with the invention. Preferred α emitters include bismuth-213, astatine-211, and radium-223. Preferred β, γ-emitters include ¹⁶⁶Ho, ¹⁸⁸Re, ¹⁸⁶Re, ¹⁵³Sm, and ⁸⁹Sr. Preferred β-emitters include ⁹⁰Y and ²²⁵Ac. Preferred γ-emitters include ⁶⁷Ga, ⁶⁸Ga, ⁶⁴Cu, ⁶¹Cu and ¹¹¹In. Preferred α-emitters include ²¹¹At and ²¹²Bi. It is also envisioned that para-magnetic substances, such as Gd, Mn, Cu or Fe can be chelated with EC for use in conjunction with the present invention.

### VIII. Kit for Preparing EC Complexes

Complexes and means for preparing such complexes are conveniently provided in a kit form including a sealed vial containing a predetermined quantity of an EC-tissue specific ligand conjugate of the invention to be labeled and a sufficient amount of reducing agent to label the conjugate with *^{99m}*Tc*. ^{99m}*Tc labeled scintigraphic imaging agents according to the present invention can be prepared by the addition of an appropriate amount of *^{99m}*Tc or *^{99m}*Tc complex into a vial containing the EC-tissue specific ligand conjugate and reducing agent and reaction under conditions described in Example 1 hereinbelow. The kit may also contain conventional pharmaceutical adjunct materials such as, for example, pharmaceutically acceptable salts to adjust the osmotic pressure, buffers, preservatives, antioxidants, and the like.

In certain embodiments, an antioxidant and a transition chelator are included in the composition to prevent oxidation of the ethylenedicysteine. In certain embodiments, the antioxidant is vitamin C (ascorbic acid). However, it is contemplated that any other antioxidant known to those of ordinary skill in the art, such as tocopherol, pyridoxine, thiamine, or rutin, may also be used. Examples of transition chelators for use in the present invention include, but are not limited to, glucoheptonate, gluconate, glucarate, citrate, and tartarate. In certain embodiments of the present invention, the transition chelator is gluconate or glucarate, as these do not interfere with the stability of ethylenedicysteine. The components of the kit may be in liquid, frozen or dry form. In a preferred embodiment, kit components are provided in lyophilized form.

### IX. Radioactively Labeled Reagents

Radioactively labeled reagents or conjugates provided by the present invention are provided having a suitable amount of radioactivity. In forming *^{99m}*Tc radioactive complexes, it is generally preferred to form radioactive complexes in solutions containing radioactivity at concentrations of from about 0.01 millicurie (mCi) to about 300 mCi per mL.

*^{99m}Tc* labeled scintigraphic imaging agents provided by the present invention can be used for visualizing sites in a mammalian body. In accordance with this invention, the *^{99m}*Tc labeled scintigraphic imaging agents are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with this invention. Generally, the unit dose to be administered has a radioactivity of about 0.01 mCi to about 300 mCi, preferably 10 mCi to about 200 mCi. The solution to be injected at unit dosage is from about 0.01 mL to about 10 mL. After intravenous administration, imaging of the organ or tumor *in vivo* can take place, if desired, in hours or even longer, after the radiolabeled reagent is introduced into a patient. In most instances, a sufficient amount of the administered dose will accumulate in the area to be imaged within about 0.1 of an hour to permit the taking of gamma scintigrams. Any conventional method of scintigraphic imaging for diagnostic or prognostic purposes can be utilized in accordance with this invention.

### X. Uses For ^{99m}Tc-EC Conjugates

The *^{99m}*Tc-EC labeling strategy of the invention may also be used for prognostic purposes. It is envisioned that EC-conjugates, listed in Table 1, may be administered to a patient having a tumor. It is envisioned that the use of *^{99m}*Tc-EC as a labeling strategy can be effective with ligands designed for targeting disease receptors, hypoxia markers, apoptosis defects, disease cell cycles, disease functional pathways, and assessment of pharmaceutical agents effectiveness of these biochemical processes. Imaging may be performed to determine the effectiveness of the *^{99m}*Tc-EC-conjugate against a patient's specific problem relating to disease receptors, hypoxia markers, apoptosis defects, disease cell cycles, disease functional pathways, and assessment of pharmaceutical agent's effectiveness on these biochemical processes. Using this methodology physicians can quickly determine which *^{99m}*Tc-EC-conjugate will be most effective for the patient and design the corresponding therapy or mode of treatment. This novel methodology represents a dramatic improvement over the current methods of choosing a drug and administering a round of chemotherapy, which may involve months of the patient's time at a substantial cost before the effectiveness of the cancer chemotherapeutic agent can be determined.

The present invention could also be used to monitor the progress of former patients who have sucessfully undergone chemotherapy or radiation treatment to determine if cancer has remained in remission or is metastasizing. People with a history of cancer in their family or who have been diagnosed with a gene(s) associated with cancer can undergo monitoring by health professionals using the methodology of the current invention. The methods and pharmaceutical agents of the current invention can also be used by a health professional to monitor if cancer has started to develop in a person with cancer risk factors due to environmental exposure to carcinogens.

### XI. Tumor Angiogenesis Targeting

Throughout this application, "tumor angiogenesis targeting" refers to the use of an agent to bind to tumor neovascularization and tumor cells. Agents that are used for this purpose are known to those of ordinary skill in the art for use in performing various tumor measurements, including measurement of the size of a tumor vascular bed, and measurement of tumor volume. Some of these agents bind to the vascular wall. One of ordinary skill in the art would be familiar with the agents that are available for use for this purpose. A tumor angiogenesis targeting ligand is a ligand that is used for the purpose of tumor angiogenesis targeting as defined above. Examples of these agents include *^{99m}*Tc-EC-celebrex (EC-COX-2), *^{99m}*Tc-EC-C225, *^{99m}*Tc-EC-herceptin, *^{99m}*Tc-EC-angiostatin, and *^{99m}*Tc-EC-thalidomide have been developed for the assessment of biochemical process on angiogenesis.

### XII. Tumor Apoptosis Targeting

"Tumor apoptosis targeting" refers to use of an agent to bind to a cell that is undergoing apoptosis or at risk of undergoing apoptosis. These agents are generally used to provide an indicator of the extent or risk of apoptosis, or programmed cell death, in a population of cells, such as a tumor. One of ordinary skill in the art would be familiar with agents that are used for this purpose. Examples of apoptosis targeting agents are shown in Table 1. A "tumor apoptosis targeting ligand" is a ligand that is capable of performing "tumor apoptosis targeting" as defined in this paragraph. The targeting ligand of the present invention may include TRAIL, which is TNF-related apoptosis inducing ligand, a member of the tumor necrosis factor ligand family that rapidly induces apoptosis in a variety of transformed cell lines. The targeting ligand of the present invention may also comprise a caspase-3 targeting ligand.

Apoptosis suppressors are targets for drug discovery, with the idea of abrogating their cytoprotective functions and restoring apoptosis sensitivity to tumor cells (Reed, 2003).

### XIII. Disease Receptor Targeting

In "disease receptor targeting," certain agents are exploited for their ability to bind to certain cellular receptors that are overexpressed in disease states, such as cancer. Examples of such receptors which are targeted include estrogen receptors, androgen receptors, pituitary receptors, transferrin receptors, and progesterone receptors. Examples of agents that can be applied in disease-receptor targeting are shown in Table 1.

The radiolabeled ligands, such as pentetreotide, octreotide, transferrin, and pituitary peptide, bind to cell receptors, some of which are overexpressed on certain cells. Since these ligands are not immunogenic and are cleared quickly from the plasma, receptor imaging would seem to be more promising compared to antibody imaging.

In this invention, the inventors developed a series of new receptor ligands. These ligands are ^{99m}Tc-EC-leuteinizing hormone (*^{99m}*Tc-EC-LH) and *^{99m}*Tc-EC-transferrin.

### XIV. Disease Cell Cycle Targeting

Gene based analogues for *in vivo* measurement of cell proliferation has been demonstrated by PET (Alauddin *et al.*, 2001).

Disease cell cycle targeting refers to targeting of agents that are upregulated in proliferating cells. Compounds used for this purpose can be used to measure various parameters in cells, such as tumor cell DNA content.

Many of these agents are nucleoside analogues. For example, pyrimidine nucleoside (*e.g*., 2'-fluoro-2'-deoxy-5-iodo-1-β-D-arabinofuranosyluracil [FIAU], 2'-fluoro-2'-deoxy-5-iodo-1-β-D-ribofuranosyl-uracil [FIRU], 2'-fluoro-2'-5-methyl-1-β-D-arabinofuranosyluracil [FMAU], 2'-fluoro-2'-deoxy-5-iodovinyl-1-β-D-ribofuranosyluracil [IVFRU]) and acycloguanosine: 9-[(2-hydroxy-1-(hydroxymethyl)ethoxy)methyl]guanine (GCV) and 9-[4-hydroxy-3-(hydroxymethyl)butyl]guanine (PCV) (Tjuvajev *et al.*, 2002; Gambhir *et al.*, 1998; Gambhir *et al.*, 1999) and other ¹⁸F-labeled acycloguanosine analogs, such as 8-fluoro-9-[(2-hydroxy-1-(hydroxymethyl)ethoxy)methyl]guanine (FGCV) (Gambhir *et al.*, 1999; Namavari *et al.*, 2000), 8-fluoro-9-[4-hydroxy-3-(hydroxymethyl)butyl]guanine (FPCV) (Gambhir *et al.*, 2000; Iyer *et al.*, 2001), 9-[3-fluoro-1-hydroxy-2-propoxy methyl]guanine (FHPG) (Alauddin *et al.*, 1996; Alauddin *et al.*, 1999), and 9-[4-fluoro-3-(hydroxymethyl)butyl]guanine (FHBG) (Alauddin and Conti, 1998; Yaghoubi *et al.*, 2001) have been developed as reporter substrates for imaging wild-type and mutant (Gambhir *et al.*, 2000) HSV1-tk expression. One or ordinary skill in the art would be familiar with these and other agents that are used for disease cell cycle targeting.

In this invention, the inventors developed a series of disease cell cycle targeting ligands. These ligands include, for example, *^{99m}*Tc-EC-adenosine and *^{99m}*Tc-EC-penciclovir (EC-guanine).

### XV. Drug Assessment

Certain drug-based ligands of the present invention can be applied in measuring the pharmacological response of a subject to a drug. A wide range of parameters can be measured in determining the response of a subject to administration of a drug. One of ordinary skill in the art would be familiar with the types of responses that can be measured. These responses depend in part upon various factors, including the particular drug that is being evaluated, the particular disease or condition for which the subject is being treated, and characteristics of the subject. Radiolabeled agents can be applied in measuring drug assessment.

In this invention, the inventors developed a new drug based ligand, *^{99m}*Tc-EC-carnitine (Taggart *et al.*, 1999). Other examples of these agents are shown in Table 1.

### XVI. Pharmaceutical Preparations

Pharmaceutical compositions of the present invention comprise an effective amount of a radiolabeled ethylenedicysteine derivative, or more particularly ^{99m}Tc-EC derivatives or additional agent dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that contains at least one radiolabeled ethylenedicysteine derivative or additional active ingredient will be known to those of skill in the art in light of the present disclosure, as exemplified by Remington's Pharmaceutical Sciences, 18th Ed. Mack Printing Company, 1990, incorporated herein by reference. Moreover, for animal (*e.g*., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329, incorporated herein by reference). Except insofar as any conventional carrier is incompatible with the active ingredient, its use in the therapeutic or pharmaceutical compositions is contemplated.

The radiolabeled ethylenedicysteine derivative may comprise different types of carriers depending on whether it is to be administered in solid, liquid or aerosol form, and whether it needs to be sterile for such routes of administration such as injection. The present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, injection, infusion, continuous infusion, localized perfusion bathing target cells directly, via a catheter, via a lavage, in lipid compositions (*e.g*., liposomes), or by other method or any combination of the forgoing as would be known to one of ordinary skill in the art (see, for example, Remington's Pharmaceutical Sciences, 18th Ed. Mack Printing Company, 1990, incorporated herein by reference).

The actual dosage amount of a composition of the present invention administered to a patient can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain embodiments, pharmaceutical compositions may comprise, for example, at least about 0.1 % of a radiolabeled ethylenedicysteine derivative. In other embodiments, the active compound may comprise between about 2% to about 75% of the weight of the unit, or between about 25% to about 60%, for example, and any range derivable therein. In other non-limiting examples, a dose may also comprise from about 0.1 mg/kg/body weight, 0.5 mg/kg/ body weight, 1 mg/kg/body weight, about 5 mg/kg/body weight, about 10 mg/kg/body weight, about 20 mg/kg/body weight, about 30 mg/kg/body weight, about 40 mg/kg/body weight, about 50 mg/kg/body weight, about 75 mg/kg/body weight, about 100 mg/kg/body weight, about 200 mg/kg/body weight, about 350 mg/kg/body weight, about 500 mg/kg/body weight, about 750 mg/kg/body weight, to about 1000 mg/kg/body weight or more per administration, and any range derivable therein. In non-limiting examples of a derivable range from the numbers listed herein, a range of about 10 mg/kg/body weight to about 100 mg/kg/body weight, etc., can be administered, based on the numbers described above.

In any case, the composition may comprise various antioxidants to retard oxidation of one or more component. Additionally, the prevention of the action of microorganisms can be brought about by preservatives such as various antibacterial and antifungal agents, including, but not limited to parabens (*e.g*., methylparabens, propylparabens), chlorobutanol, phenol, sorbic acid, thimerosal or combinations thereof.

The radiolabeled ethylenedicysteine derivative may be formulated into a composition in a free base, neutral or salt form. Pharmaceutically acceptable salts include the salts formed with the free carboxyl groups derived from inorganic bases such as for example, sodium, potassium, ammonium, calcium or ferric hydroxides; or such organic bases as isopropylamine, trimethylamine, histidine or procaine.

In embodiments where the composition is in a liquid form, a carrier can be a solvent or dispersion medium comprising, but not limited to, water, ethanol, polyol (*e.g*., glycerol, propylene glycol, liquid polyethylene glycol, *etc.*), lipids (*e.g*., triglycerides, vegetable oils, liposomes) and combinations thereof. The proper fluidity can be maintained, for example, by the use of a coating, such as lecithin; by the maintenance of the required particle size by dispersion in carriers such as, for example liquid polyol or lipids; by the use of surfactants such as, for example hydroxypropylcellulose; or combinations thereof such methods. In many cases, it will be preferable to include isotonic agents, such as, for example, sugars, sodium chloride or combinations thereof.

Sterile injectable solutions are prepared by incorporating the radiolabeled ethylenedicysteine derivative in the required amount of the appropriate solvent with various amounts of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

In particular embodiments, prolonged absorption of an injectable composition can be brought about by the use in the compositions of agents delaying absorption, such as, for example, aluminum monostearate, gelatin or combinations thereof.

### XVII. Combinational Therapy

It is an aspect of this invention that the radiolabeled ethylenedicysteine derivative can be used in combination with another agent or therapy method, preferably another cancer treatment. The radiolabeled ethylenedicysteine derivative may precede or follow the other agent treatment by intervals ranging from minutes to weeks. In embodiments where the other agent and expression construct are applied separately to the cell, one would generally ensure that a significant period of time did not expire between the time of each delivery, such that the agent and expression construct would still be able to exert an advantageously combined effect on the cell. For example, in such instances, it is contemplated that one may contact the cell, tissue or organism with two, three, four or more modalities substantially simultaneously (*i.e*., within less than about a minute) with the radiolabeled ethylenedicysteine derivative. In other aspects, one or more agents may be administered within about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, to about 48 hours or more prior to and/or after administering the radiolabeled ethylenedicysteine derivative. In certain other embodiments, an agent may be administered within of from about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20, to about 21 days prior to and/or after administering the radiolabeled ethylenedicysteine derivative. In some situations, it may be desirable to extend the time period for treatment significantly, however, where several weeks (*e.g*., about 1, about 2, about 3, about 4, about 5, about 6, about 7 or about 8 weeks or more) lapse between the respective administrations.

Various combinations may be employed, the radiolabeled ethylenedicysteine derivative is "A" and the secondary agent, which can be any other therapeutic agent, is "B":
A/B/A B/A/B B/B/A A/A/B AB/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the therapeutic expression constructs of the present invention to a patient will follow general protocols for the administration of chemotherapeutics, taking into account the toxicity, if any, of the vector. It is expected that the treatment cycles would be repeated as necessary. It also is contemplated that various standard therapies, as well as surgical intervention, may be applied in combination with the described arsenical agent. These therapies include but are not limited to chemotherapy, radiotherapy, immunotherapy, gene therapy and surgery.

### a. Chemotherapy

Cancer therapies also include a variety of combination therapies with both chemical and radiation based treatments. Combination chemotherapy include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, cisplatinum, 5-fluorouracil, vincristin, vinblastin and methotrexate, or any analog or derivative variant of the foregoing.

### b. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells. The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### c. Immunotherapy

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionucleotide, ricin A chain, cholera toxin, pertussis toxin, *etc.*) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy, thus, could be used as part of a combined therapy, in conjunction with gene therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155.

### d. Genes

In yet another embodiment, the secondary treatment is a secondary gene therapy in which a therapeutic polynucleotide is administered before, after, or at the same time a first therapeutic agent. Delivery of the therapeutic agent in conjunction with a vector encoding a gene product will have a combined anti-hyperproliferative effect on target tissues.

### e. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies. Curative surgery includes resection in which all or part of cancerous tissue is physically or partially removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present invention may be used in conjunction with removal of superficial cancers, precancers, or incidental amounts of normal tissue.

### XVIII. Synthesis of EC

EC was prepared in a two-step synthesis according to the previously described methods (Ratner and Clarke, 1937; Blondeau *et al.*, 1967; each incorporated herein by reference). The precursor, L-thiazolidine-4-carboxylic acid, was synthesized (m.p. 195°, reported 196-197°). EC was then prepared (m.p. 237°, reported 251-253°). The structure was confirmed by ¹H-NMR and fast-atom bombardment mass spectroscopy (FAB-MS).

### XIX. Scintigraphic Imaging and Autoradiography Studies

Scintigraphic images, using a gamma camera (Siemens Medical Systems, Inc., Hoffman Estates, IL, or Philips Medical Systems, Skylight, Milpitas, CA) equipped with low-energy, parallel-hole collimator, were obtained 0.5, 2 and 4 hrs after i.v. injection of 18.5 MBq of *^{99m}*Tc-labeled radiotracer.

Whole-body autoradiogram were obtained by a quantitative image analyzer (Cyclone Storage Phosphor System, Packard, Meridian, CI.). Following i.v. injection of 37 MBq of ^{99m}Tc-EC-folate, animal killed at 1 h and body was fixed in carboxymethyl cellulose (4%). The frozen body was mounted onto a cryostat (LKB 2250 cryomicrotome) and cut into 100 µm coronal sections. Each section was thawed and mounted on a slide. The slide was then placed in contact with multipurpose phosphor storage screen (MP, 7001480) and exposed for 15 h *^{99m}*Tc-labeled). The phosphor screen was excited by a red laser and resulting blue light that is proportional with previously absorbed energy was recorded.

### XX. Definitions

As used herein the term "radionuclide" is defined as a radioactive nuclide (a species of atom able to exist for a measurable lifetime and distinguished by its charge, mass, number, and quantum state of the nucleus) which, in specific embodiments, disintegrates with emission of corpuscular or electromagnetic radiation. The term may be used interchangeably with the term "radioisotope".

The term "therapeutic agent" as used herein is defined as an agent which provides treatment for a disease or medical condition. The agent in a specific embodiment improves at least one symptom or parameter of the disease or medical condition. For instance, in tumor therapy, the therapeutic agent reduces the size of the tumor, inhibits or prevents growth or metastases of the tumor, or eliminates the tumor. Examples include a drug, such as an anticancer drug, a gene therapy composition, a radionuclide, a hormone, a nutriceutical, or a combination thereof.

The term "tumor" as used herein is defined as an uncontrolled and progressive growth of cells in a tissue. A skilled artisan is aware other synonymous terms exist, such as neoplasm or malignancy. In a specific embodiment, the tumor is a solid tumor. In other specific embodiments, the tumor derives, either primarily or as a metastatic form, from cancers such as of the liver, prostate, pancreas, head and neck, breast, brain, colon, adenoid, oral, skin, lung, testes, ovaries, cervix, endometrium, bladder, stomach, and epithelium (such as a wart).

The term "drug" as used herein is defined as a compound which aids in the treatment of disease or medical condition or which controls or improves any physiological or pathological condition associated with the disease or medical condition. In a specific embodiment, the drug is a ^{99m}Tc-EC-drug conjugate.

The term "anticancer drug" as used herein is defined as a drug for the treatment of cancer, such as for a solid tumor. The anticancer drug preferably reduces the size of the tumor, inhibits or prevents growth or metastases of the tumor, and/or eliminates the tumor.

As used herein the specification, "a" or "an" may mean one or more. As used herein in the claim(s), when used in conjunction with the word "comprising", the words "a" or "an" may mean one or more than one. As used herein "another" may mean at least a second or more.

### EXAMPLE 1: TARGETING INTEGRIN (αᵥβ₃) WITH EC-ANGIOSTATIN

### a. Synthesis of Ethylenedicysteine (EC)

Integrin (αᵥβ₃) is a disease angiogenic target that can be targeted with EC-angiostatin, a compound of the present invention. EC was prepared in a two-step synthesis according to the previously described methods (Ratner and Clarke, 1937; Blondeau *et al.*, 1967; each incorporated herein by reference). The precursor, L-thiazolidine-4-carboxylic acid, was synthesized (m.p. 195°, reported 196-197°). EC was then prepared (m.p. 237°, reported 251-253°). The structure was confirmed by 1H-NMR and fast-atom bombardment mass spectroscopy (FAB-MS).

### b. Radiosynthesis of ^{99m}Tc-EC-Angiostatin

Sodium bicarbonate (1N, 1 ml) was added to a stirred solution of EC (3.27 mg, 12.2 mmol). To this colorless solution, sulfo-N-hydroxysulfosuccinimide (Sulfo-NHS, Pierce Chemical Co., Radford, IL) (3.0 mg, 13.8 µmol) and 1-ethyl-3-(3-dimethylaminopropyl) carbodiimide (EDC, 2.0 mg, 10.5 µmol) (Aldrich Chemical Co., Milwaukee, WI) were added. Angiostatin (32.7 mg, 0.93 µmol) was then added. The mixture was stirred at room temperature for 18 hrs. The mixture was dialyzed for 48 hrs (M.W. cut-off of 10,000). EC-angiostatin weighed 17mg (yield: 100%) after freeze drying. *^{99m}*Tc-pertechnetate (5.5 mCi) (Syncor Pharmaceutical Inc., Houston, TX) was added to a vial containing the lyophilized residue of EC-angiostatin (10 µg, 0.5 nmol) and tin chloride (II) (SnCl₂, 100 g, 0.53 µmol) in 0.2 ml water. The product was purified by using a sephadex G-25 column (bed volume 10 ml) (Sigma Chemical Company, St. Louis, MO) and eluted with PBS (5 ml). One milliliter of eluant was collected in each test tube. The product was isolated in Tubes 3 and 4, and yielded 3.9 mCi (70%). Radiochemical purity was assessed by Radio-TLC scanner (Bioscan, Washington, DC) using 1M ammonium acetate : methanol (4:1) as an eluant. High performance liquid chromatograph (HPLC), equipped with a GPC column (Biosep SEC-S3000, 7.8 x 300mm, Phenomenex, Torrance, CA) and two detectors (NaI and UV), was used to analyze the purity of the product. The eluant was 0.1% LiBr in PBS (10mM) and the flow rate was 1.0 ml/min.

### c. In vitro Cellular Uptake Assays and Tissue Distribution Studies

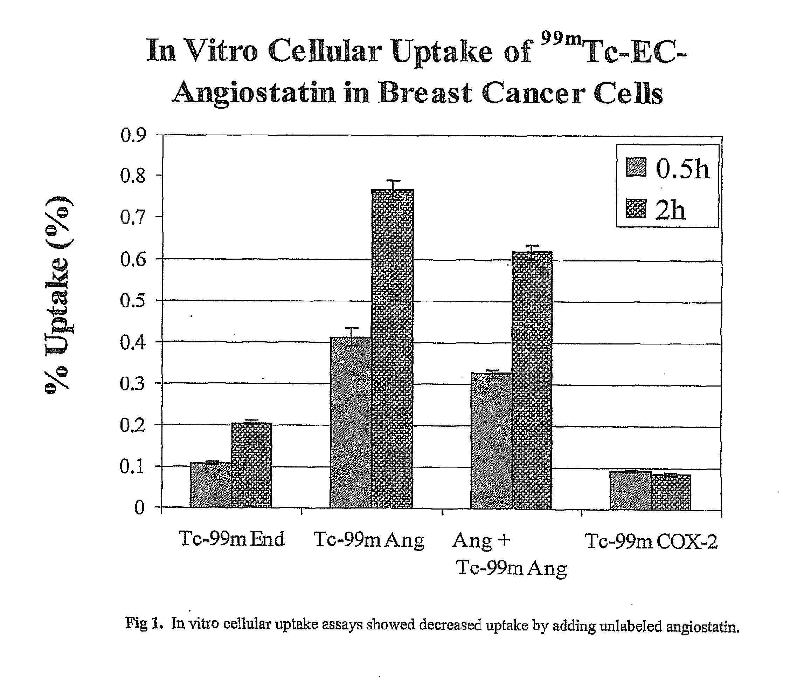

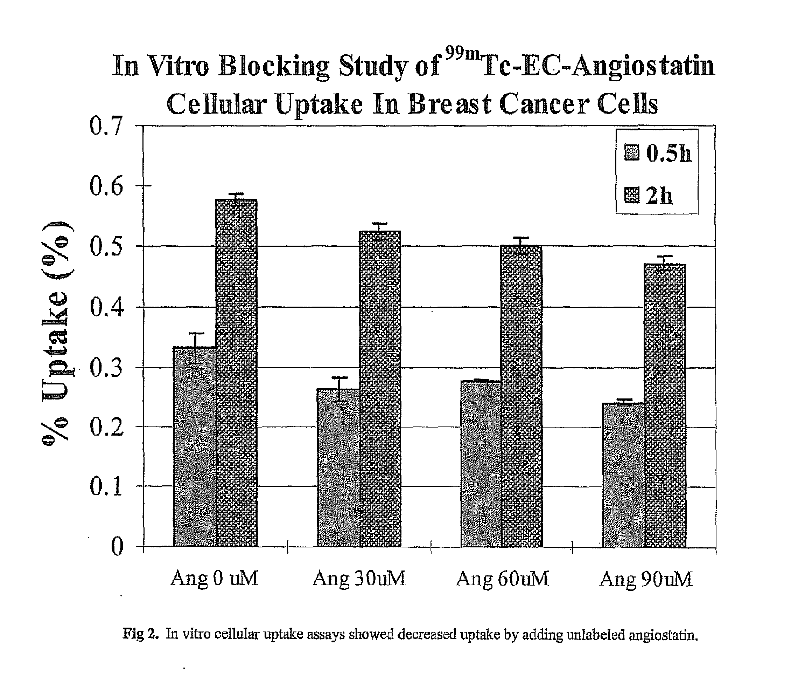

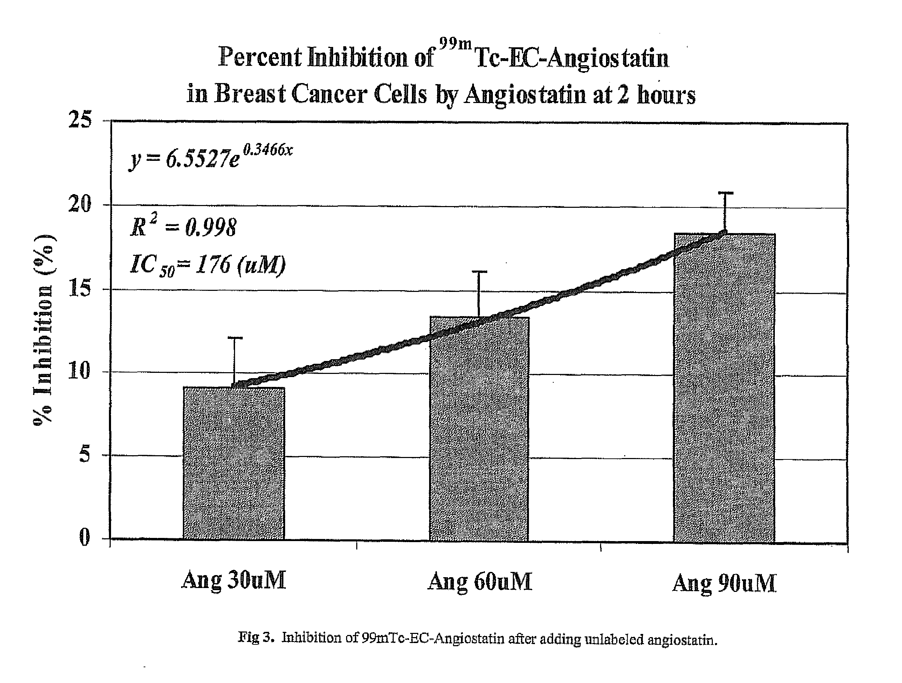

*In vitro* cell culture at various concentration of angiostatin (0-200 µM) was incubated with *^{99m}*Tc-EC-angiostatin (4 µCi/50,000 cells/well) at 0.5-2 hrs in RBA CRL-1747. There was a significant decreased uptake of *^{99m}*Tc-EC-angiostatin after adding unlabeled angiostatin in cancer cells, suggesting receptor mechanism of the uptake (FIGS. 1-2). The IC-50 of angiostatin was 176 µM (R²=0.998) (FIG. 3).

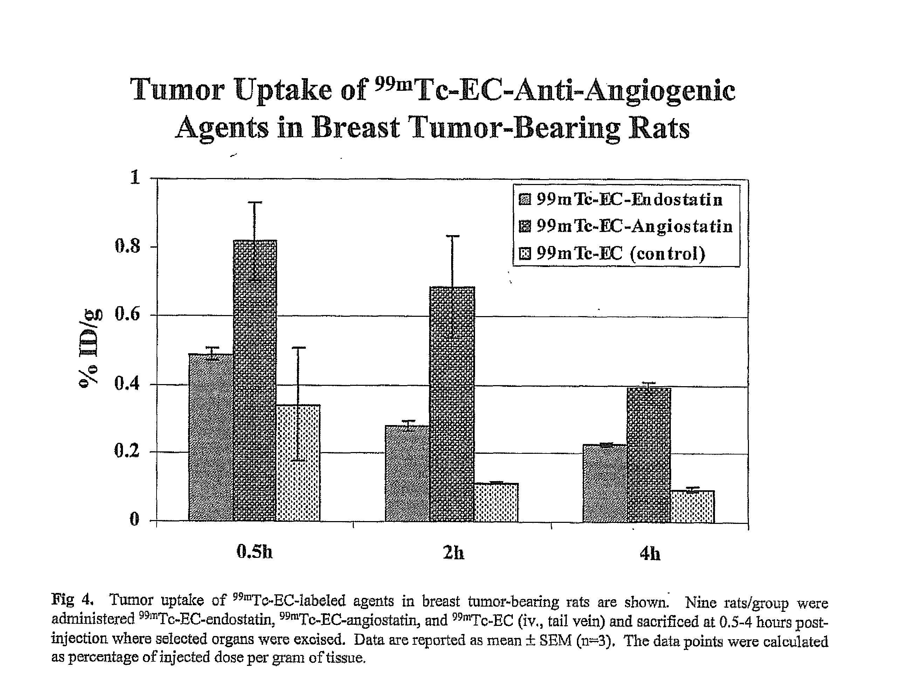

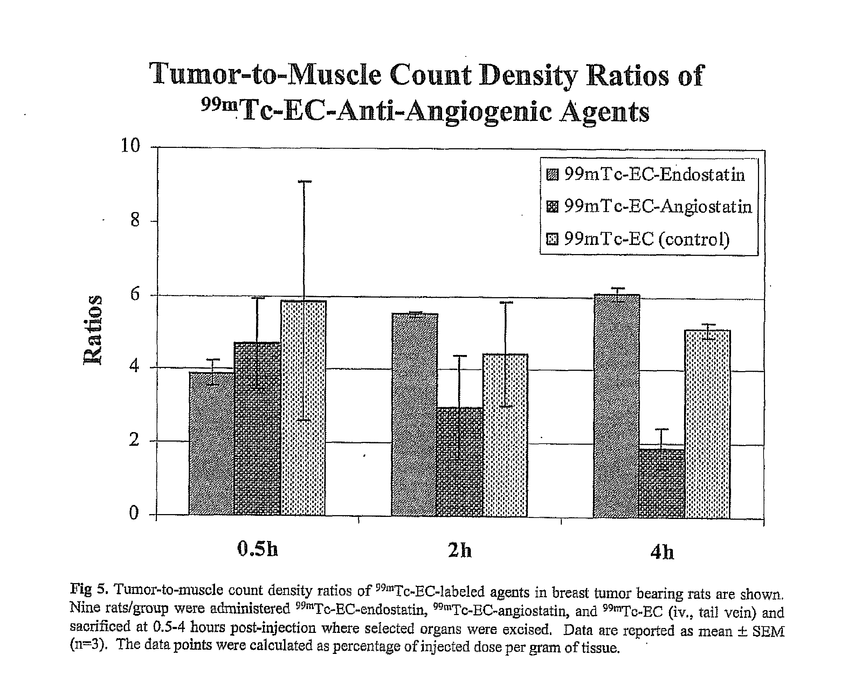

Biodistribution was assessed in mammary tumor-bearing rats (RBA CRL-1747, n=3/time interval, iv). Studies were performed 14 to 17 days after implantation when tumors reached approximately 1cm in diameter. Following administration of the radiotracer, rats were sacrificed at 0.5-4 hrs. The selected tissues were excised, weighed and counted for radioactivity by using a gamma counter (Packard Instruments, Downers Grove, IL). The biodistribution of tracer in each sample was calculated as percentage of the injected dose per gram of tissue wet weight (%ID/g). Biodistribution of *^{99m}*Tc-EC-angiostatin in tumor-bearing rats showed increased tumor-to-tissue count density ratios as a function of time (FIGS. 4-5).

### d. Scintigraphic Imaging Studies

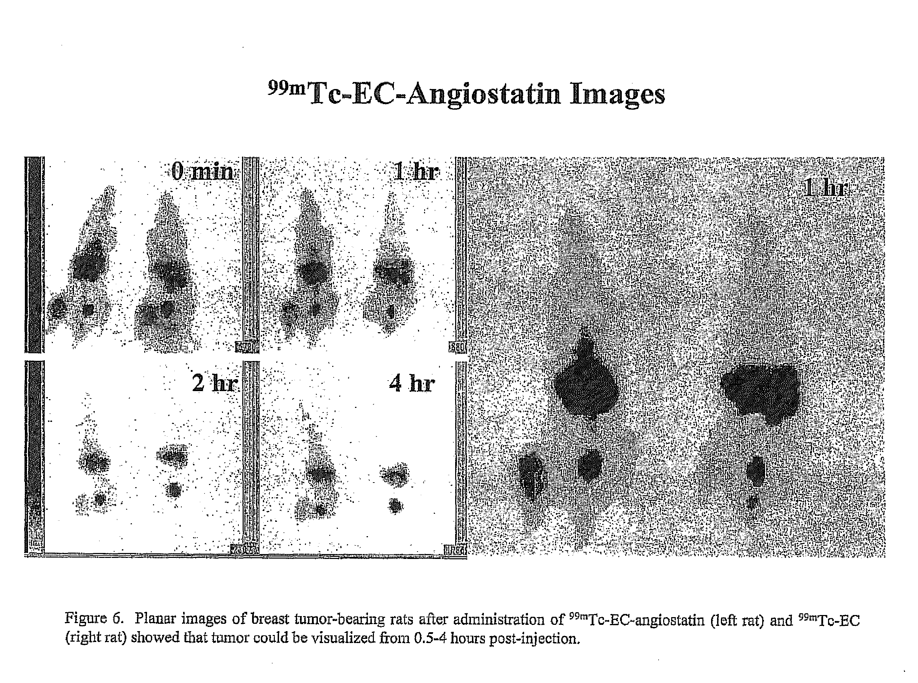

Scintigraphic imaging studies was performed in mammary tumor-bearing rats at 0.5-4 hrs (0.3 mCi/rat, n=3, iv). Control group was administered *^{99m}*Tc-EC. Computer-outlined region of interest was used to determine tumor uptake (counts/pixel) and tumor/nontumor count density ratios. Planar images confirmed that the tumors could be visualized clearly with radiolabeled angiostatin (FIG. 6).

Numerous other proteins and peptides can be applied using this technology described in Example 1. These include EC-VEGF (Vascular endothelial growth factor), EC-endostatin (anti-endothelial cells), EC-interferon α (anti-FGF).

### EXAMPLE 2: TARGETING EGFR WITH EC-C225 MONOCLONAL ANTIBODY

### a. Radiosynthesis of ^{99m}Tc-EC-C225

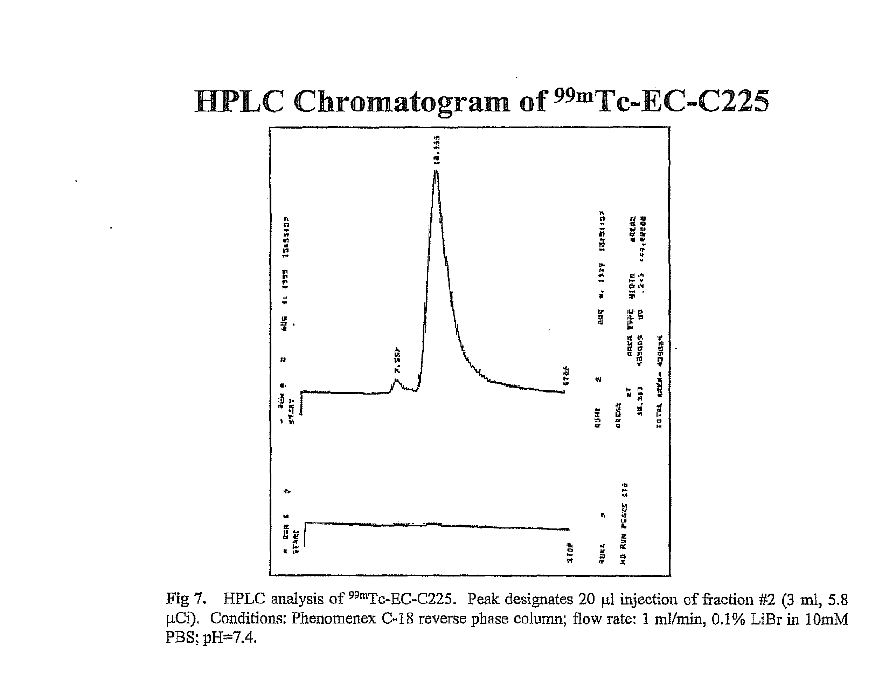

EGFR is a disease angiogenic target that can be targeted with *^{99m}*Tc-EC-C225 monoclonal antibody, a compound of the present invention. Clinical grade anti-EGF receptorR MAb C225 (IMC-C225) was obtained from ImClone Systems, Inc. (Somerville, NJ). C225 (20 mg) was stirred with EC (28.8 mg, 0.11 mmol in 1.4 ml of IN NaHCO₃), sulfo-NHS (23.3 mg, 0.11 mmol) and EDC (16.6 mg, 0.09 mmol). After dialysis, 17 mg of EC-C225 was obtained. 100 mCi of Na*^{99m}*TcO4 was added into a vial containing 1 mg EC-C225 and 100 µg SnCl₂ and the product was purified with a G-25 column and eluted with PBS, yielded 80 mCi *^{99m}*Tc-EC-C225. Radiochemical purity for *^{99m}*Tc-EC-C225 was 100% (HPLC, gel permeation column, 0.1% LiBr in 10mM PBS, pH 7.4). Specific activity was 2 Ci/µmol (FIG. 7).

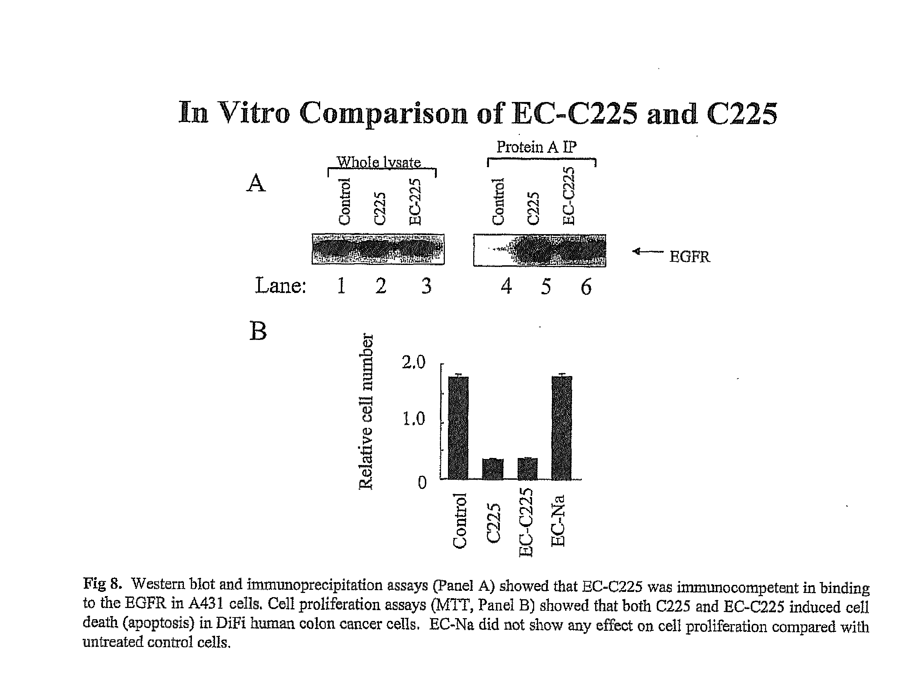

For example, an immunoassay (Western Blot and Immunoprecipitation) and cell proliferation assays were used to examine the integrity of EC-C225. Briefly, DiFi cells are known to undergo apoptosis when they are exposed to C225 in cell culture. Cells were seeded onto 24-well culture plates. Cell viability was assayed by adding 50 ul of 10 mg/ml MTT (3-[4,5-dimethylthiazol-2-yl]-2,5-diphenyltetrazolium bromide) (Sigma) into 0.5 ml of culture medium and the cells were incubated for 3 h at a 37 °C in a CO₂ incubator, followed by cell lysis with buffer containing 20% SDS in dimethyl formamide/H₂O, pH 4.7, at 37°C for more than 6 h. Cell viability was then determined by measuring the optical absorbance of cell lysate at a wavelength of 595 nm and normalizing the value with the corresponding untreated cells. The result shows that EC-C225 is biologically active in inducing cell death in DiFi cells. In contrast, the EC-Na did not show any effect on cell proliferation when compared with untreated control cells (FIG. 8).

### b. Tissue Distribution Studies

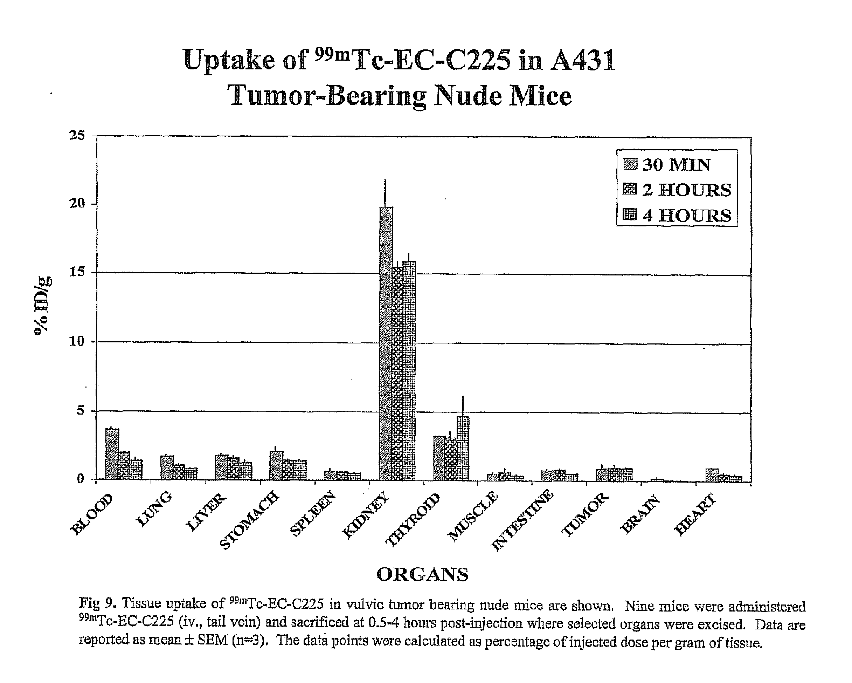

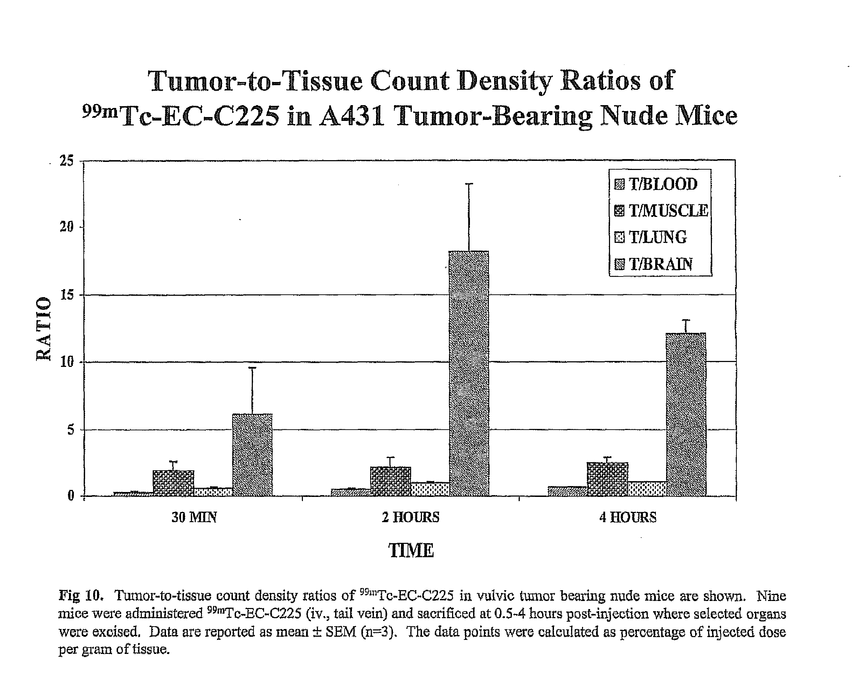

Biodistribution was assessed in A431 (an EGFR overexpressing xenograft) grown in nude mice (1µCi/mouse, 10 µg/mouse, n=3/time interval, iv). Studies were performed 14 to 17 days after implantation when tumors reached approximately 1cm in diameter. Following administration of the radiotracer, mice were sacrificed at 0.5-4 hrs. The selected tissues were excised, weighed and counted for radioactivity by using a gamma counter. The biodistribution of tracer in each sample was calculated as percentage of the injected dose per gram of tissue wet weight (%ID/g). Biodistribution studies showed that tumor-to muscle count density ratios increased as a function of time (FIG. 9). Tumor uptake (%ID/g) was 1±0.2%. Total tumor uptake was 6% of injected dose (FIG. 10).

### c. Scintigraphic Imaging Studies

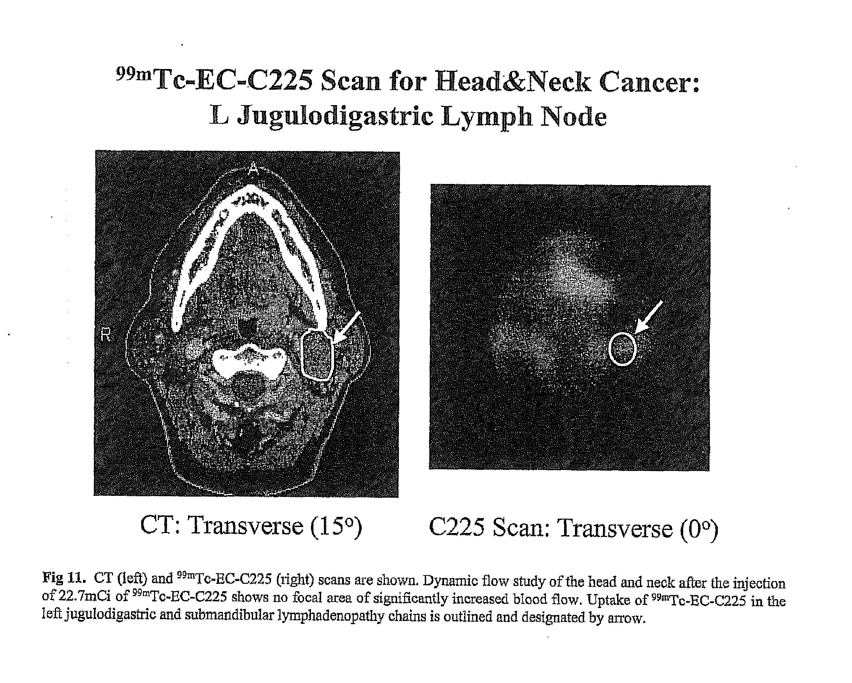

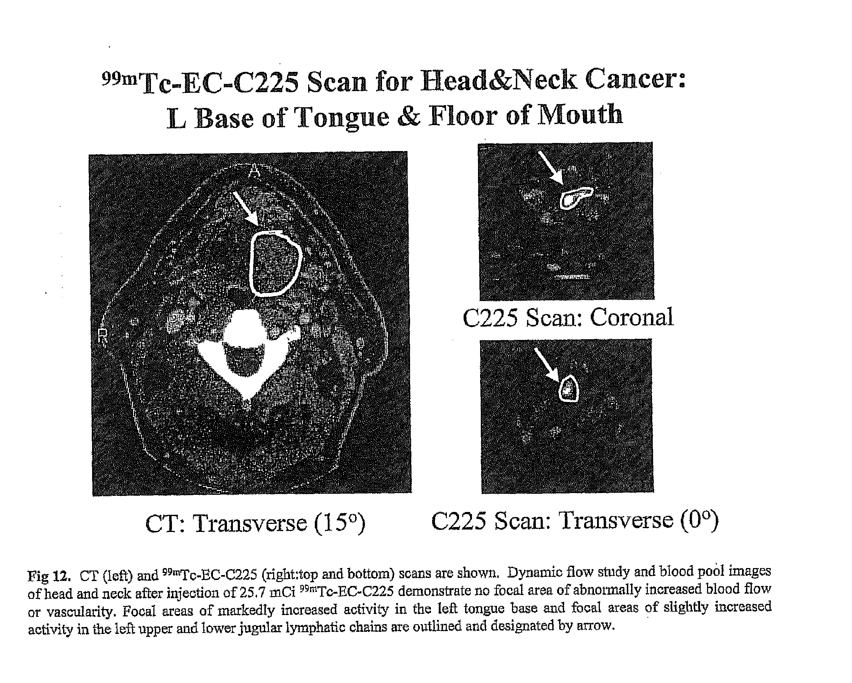

SPECT imaging (25-30 mCi/patient) was performed in 5 patients with HNSCC at 0.5-24 hrs. Each patient underwent a scan prior to C225 antibody therapy in combination with radiotherapy. Computer-outlined region of interest was used to determine tumor uptake (counts/pixel) and tumor/nontumor count density ratios. Clinical SPECT images showed that tumor could be visualized at 0.5-4 hrs. Images and findings were illustrated in FIGS. 11-12.

Other antibodies can be applied using the EC technology, including EC-Herceptin (anti-HER2), EC-CD31, EC-CD40 (immunomodulator), EC-HSA (human serum albumin).

### EXAMPLE 3: TARGETING COX-2 ENZYME WITH EC-COX-2

### a. Synthesis of Ethylamino COX-2 (EA- COX-2)

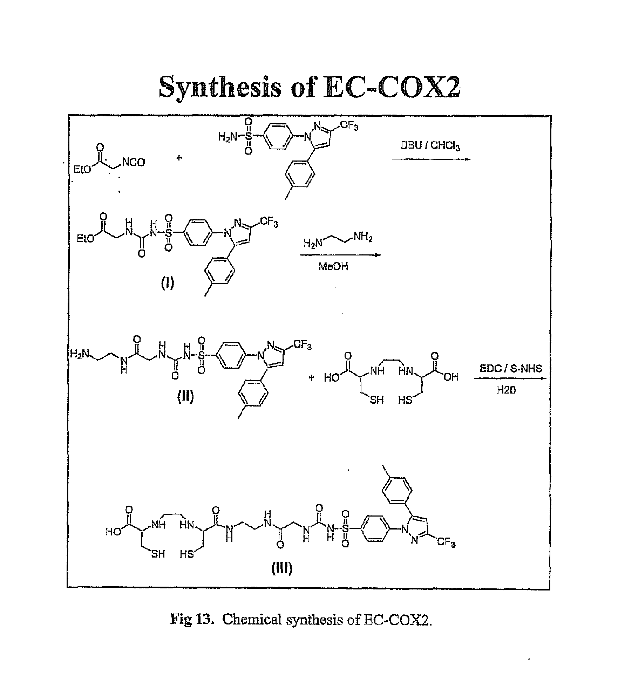

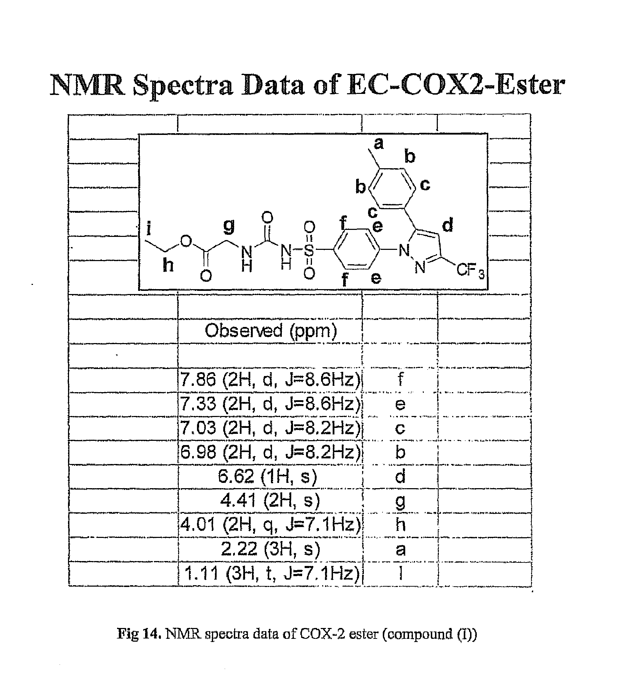

The COX-2 enzyme is a disease angiogenic target that can be targeted with EC-COX-2, a compound of the present invention. N-4-(5-p-tolyl-3-trifluoromethyl-pyrazol-1-yl)benzenesulfonylamide (COX-2 inhibitor) (114.4mg, 0.3mmol) was dissolved in chloroform (2ml). To this solution, DBU 44.9 µl (0.3mmol in chloroform 0.5ml) and ethyl isocyanatoacetate 33.7 µl (0.3mmol in chloroform 0.5ml) were added. The reaction was stirred at room temperature for 6 hours. The solvent was evaporated under vacuo. The product was isolated from silica gel-packed column using chloroform/methanol as an eluant. The yield of the ester form of COX-2 (compound I) was 135mg (88.1%). The synthetic scheme is shown in FIG. 13. NMR spectra data was recorded in FIG. 14.

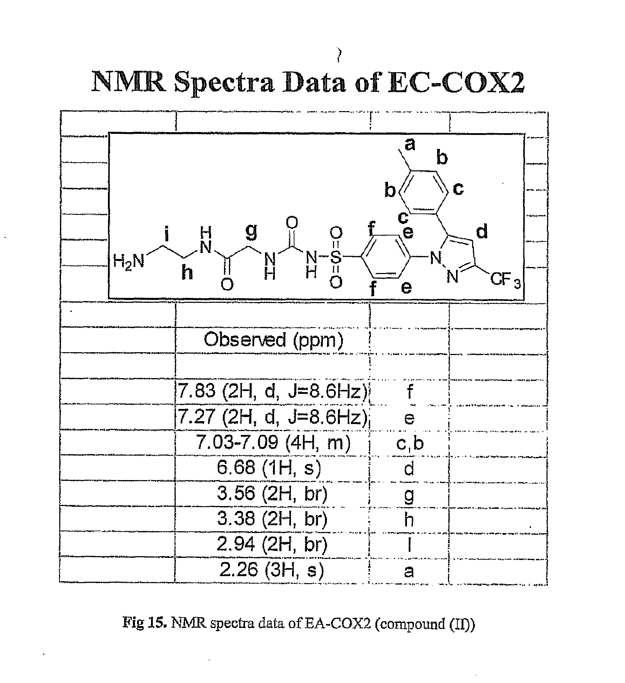

Compound I (102mg, 0.2mmol) was dissolved in 2ml of methanol and ethylene diamine (72.9 µl) was added. The reaction was stirred at room temperature for 24 hours. The product was isolated from silica gel-packed column using chloroform/methanol as an eluant. The desired ethylamino COX-2 (EA- COX-2) (compound II) was isolated (91mg, 86.7% yield). NMR spectra data of compound II was recorded in FIG. 15.

### b. Synthesis of EC-ethylamino COX-2 (EC- COX-2)

To dissolve EC, NaOH (1N, 0.6 ml) was added to a stirred solution of EC (42.3 mg, 0.15 mmol) in water (3 ml). To this colorless solution, sulfo-NHS (65.1 mg, 0.3 mmol) and EDC (57.5 mg, 0.3 mmol) were added. EA-COX-2 (78.6 mg, 0.15 mmol) was then added. The mixture was stirred at room temperature for 24 hours and then dialyzed for 48 hours using Spectra/POR molecular porous membrane with molecule cut-off at 500 (Spectrum Medical Industries Inc., Houston, TX). After dialysis, the product was freeze dried. The product weighed 87.5 mg (yield 75%).

### c. Radiolabeling of EC-COX-2 with ^{99m}Tc

Radiosynthesis of *^{99m}*Tc- EC-COX-2 was achieved by adding required amount of *^{99m}*Tc-pertechnetate into home-made kit containing the lyophilized residue of EC-COX-2 (5 mg), SnCl₂ (100 µg), Na₂HPO₄ (13.5 mg), ascorbic acid (0.5 mg), glutamic acid (2 mg) and EC (0.5 mg). Final pH of preparation was 7.4. Radiochemical purity was determined by TLC (ITLC SG, Gelman Sciences, Ann Arbor, MI) eluted with, respectively ammonium acetate (1M in water) : methanol (4:1). From radio-TLC (Bioscan, Washington, DC) analysis, the radiochemical purity was >95 %.

### d. In Vitro Cellular Uptake Assays and Tissue Distribution Studies

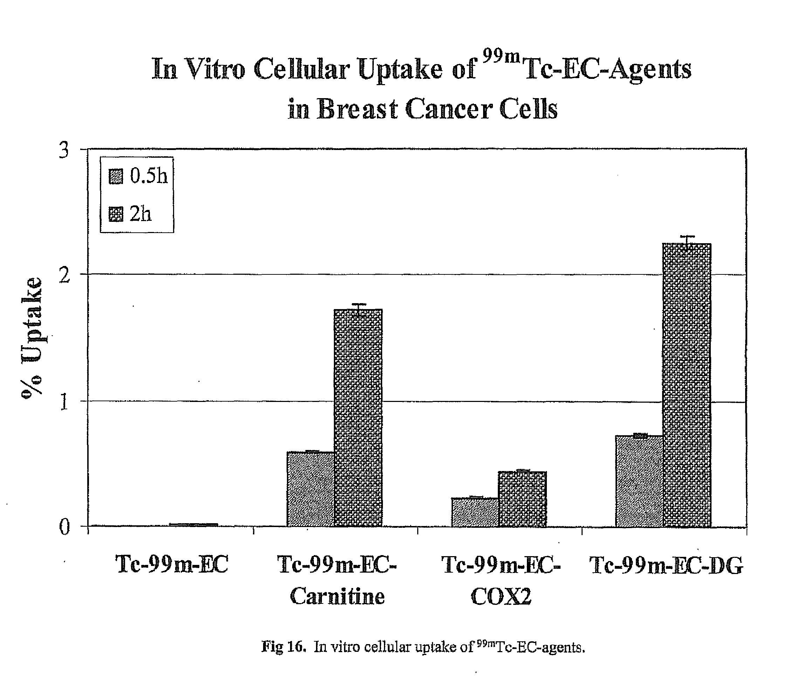

An *in vitro* cell culture using tumor cells was incubated with *^{99m}*Tc-EC-COX-2 (4 µCi/50,000 cells/well) at 0.5-2 hrs in RBA CRL-1747. There was a significant increase in uptake compared to *^{99m}*Tc-EC (FIGS. 1 and 16).

Biodistribution was assessed in mammary tumor-bearing rats (RBA CRL-1747, n=3/time interval, iv). Studies were performed 14 to 17 days after implantation when tumors reached approximately 1cm in diameter. Following administration of the radiotracer, rats were sacrificed at 0.5-4 hrs. The selected tissues were excised, weighed and counted for radioactivity by using a gamma counter (Packard Instruments, Downers Grove, IL). The biodistribution of tracer in each sample was calculated as percentage of the injected dose per gram of tissue wet weight (% ID/g). Biodistribution of *^{99m}*Tc-EC-COX-2 in tumor-bearing rats showed increased tumor-to-tissue count density ratios as a function of time compared to *^{99m}*Tc-EC (Tables 2 and 3).

**TABLE 2: Biodistribution of ^{99m}Tc-EC in Breast Tumor Bearing Rats**

| | 30 Min. | 1 Hour | 2 Hour | 4 Hour |
|---|---|---|---|---|
| Blood | 0.435±0.29 | 0.273±0.039 | 0.211±0.001 | 0.149±0.008 |
| Lung | 0.272±0.019 | 0.187±0.029 | 0.144±0.002 | 0.120±0.012 |
| Liver | 0.508±0.062 | 0.367±0.006 | 0.286±0.073 | 0.234±0.016 |
| Stomach | 0.136±0.060 | 0.127±0.106 | 0.037±0.027 | 0.043±0.014 |
| Kidney | 7.914±0.896 | 8.991±0.268 | 9.116±0.053 | 7.834±1.018 |
| Thyroid | 0.219±0.036 | 0.229±0.118 | 0.106±0.003 | 0.082±0.005 |
| Muscle | 0.060±0.006 | 0.043±0.002 | 0.028±0.009 | 0.019±0.001 |
| Intestine | 0.173±0.029 | 0.787±0.106 | 0.401±0.093 | 0.103±0.009 |
| Urine | 9.124±0.808 | 11.045±6.158 | 13.192±4.505 | 8.693±2.981 |
| Tumor | 0.342±0.163 | 0.149±0.020 | 0.115±0.002 | 0.096±0.005 |
| Tumor/Blood | 0.776±0.322 | 0.544±0.004 | 0.546±0.010 | 0.649±0.005 |
| Tumor/Muscle | 5.841±3.253 | 3.414±0.325 | 4.425±1.397 | 5.093±0.223 |
| Tumor/Lung | 1.256±0.430 | 0.797±0.022 | 0.797±0.002 | 0.798±0.007 |

Values shown represent the mean ± standard deviation of data from 3 animals.

**TABLE 3: Biodistribution of ^{99m}Tc-EC-COX-2 in Breast Tumor-Bearing Rats % of injected dose per gram of tissue weight (n=3/time, interval,iv)**

| **30 min** | | | **2h** | | **4h** | |
|---|---|---|---|---|---|---|
| BLOOD | 2.293 | ±0.038 | 1.388 | ±0.063 | 1.031 | ±0.033 |
| HEART | 0.475 | ±0.025 | 0.283 | ±0.017 | 0.224 | ±0.004 |
| LUNG | 1.033 | ±0.035 | 0.712 | ±0.098 | 0.500 | ±0.011 |
| LIVER | 1.556 | ±0.046 | 1.461 | ±0.049 | 1.506 | ±0.080 |
| SPLEEN | 0.594 | ±0.298 | 0.965 | ±0.056 | 0.981 | ±0.041 |
| KIDNEY | 4.963 | ±0.147 | 6.088 | ±0.305 | 6.363 | ±0.260 |
| INTESTINE | 0.406 | ±0.039 | 0.276 | ±0.061 | 0.190 | ±0.006 |
| UTERUS | 0.595 | ±0.003 | 0.334 | ±0.034 | 0.263 | ±0.005 |
| MUSCLE | 0.133 | ±0.007 | 0.062 | ±0.003 | 0.002 | ±0.004 |
| TUMOR | 0.587 | ±0.062 | 0.424 | ±0.019 | 0.406 | ±0.004 |
| THYROID | 0.784 | ±0.090 | 0.449 | ±0.015 | 0.372 | ±0.021 |
| STOMACH | 0.370 | ±0.010 | 0.187 | ±0.004 | 0.139 | ±0.004 |
| BONE & JOINT | 0.324 | ±0.036 | 0.190 | ±0.003 | 0.178 | ±0.022 |
| TUMOR/ | 4.375 | ±0.304 | 6.876 | ±0.704 | 9.715 | ±0.387 |
| MUSCLE | | | | | | |
| TUMOR/ | 0.255 | ±0.022 | 0.307 | ±0.018 | 0.395 | ±0.014 |
| BLOOD | | | | | | |
| UTERUS/ | 0.259 | ±0.003 | 0.240 | ±0.021 | 0.255 | ±0.005 |
| BLOOD | | | | | | |
| UTERUS/ | 4.471 | ±0.257 | 5.442 | ±0.852 | 6.278 | ±0.199 |
| MUSCLE | | | | | | |
| BONE/ | 2.419 | ±0.225 | 3.058 | ±0.114 | 4.314 | ±0.739 |
| MUSCLE | | | | | | |

Values shown represent the mean ±standard deviation of data from 3 animals.

### e. Scintigraphic Imaging Studies

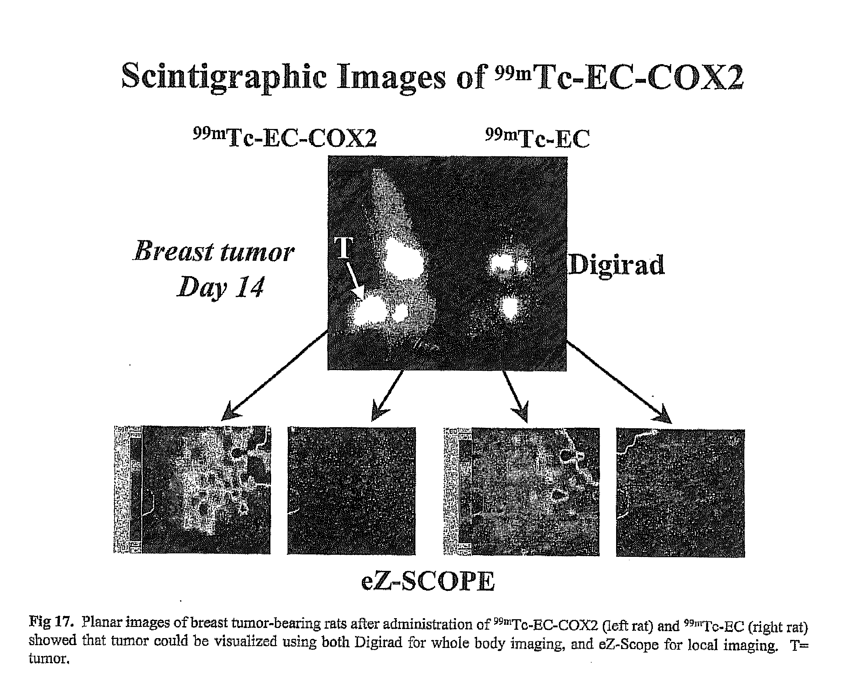

Scintigraphic imaging studies was performed in mammary tumor-bearing rats at 0.5-4 hrs (0.3 mCi/rat, n=3, iv). Control group was administered *^{99m}*Tc-EC. Planar images confirmed that the tumors could be visualized clearly with *^{99m}*Tc-EC-COX-2 (FIG. 17).

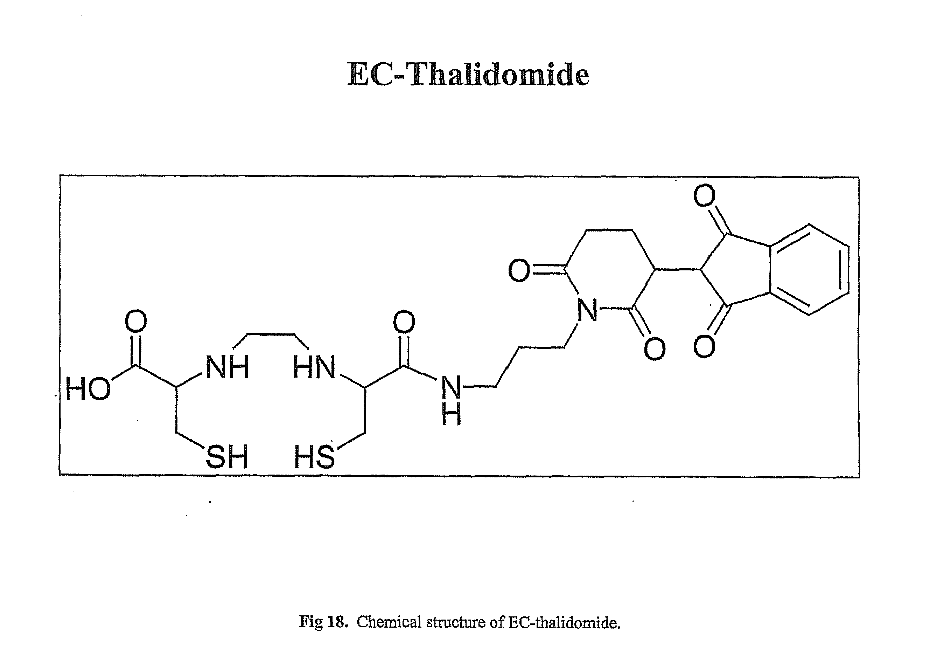

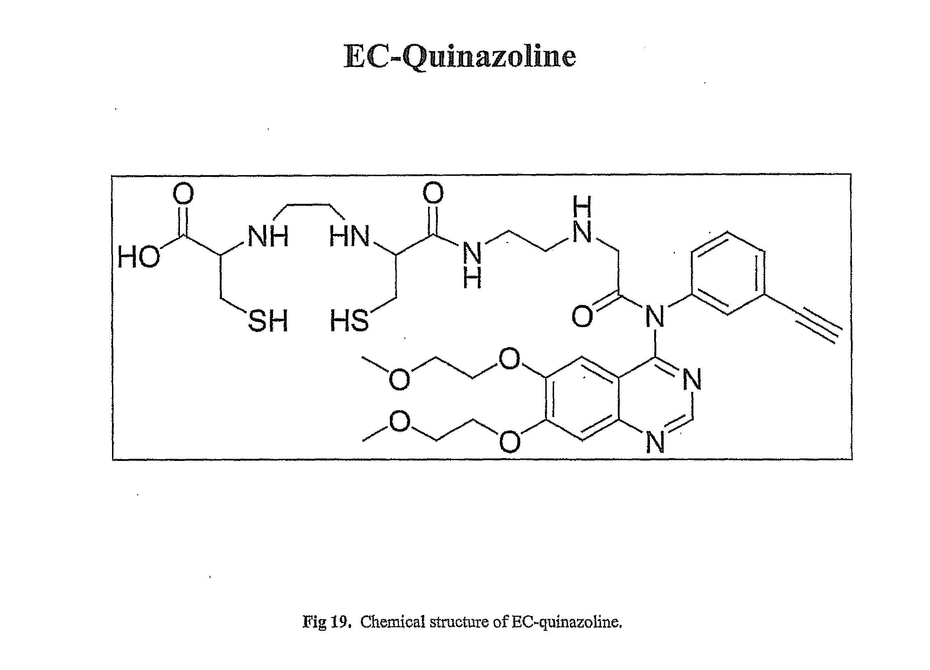

Other small molecules can be applied using the EC technology, including EC-thalidomide (anti-VEGF), EC-quinazolin analogue (anti-EGF receptorR). The structures are shown in FIGS. 18-19.

### EXAMPLE 4: TARGETING CHROMATIN WITH EC-PENCICLOVIR

### a. Synthesis of EC-Aminopenciclovir

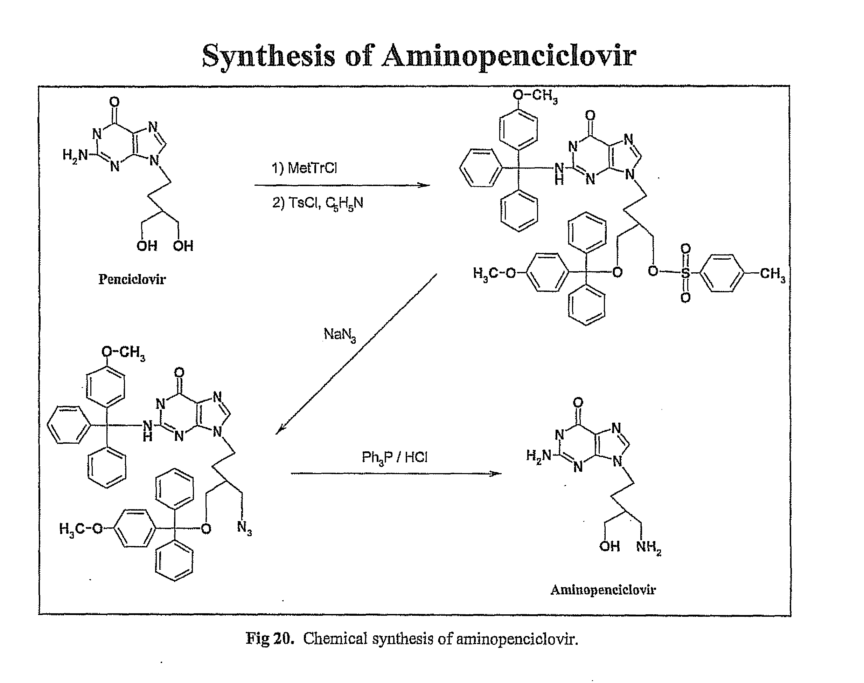

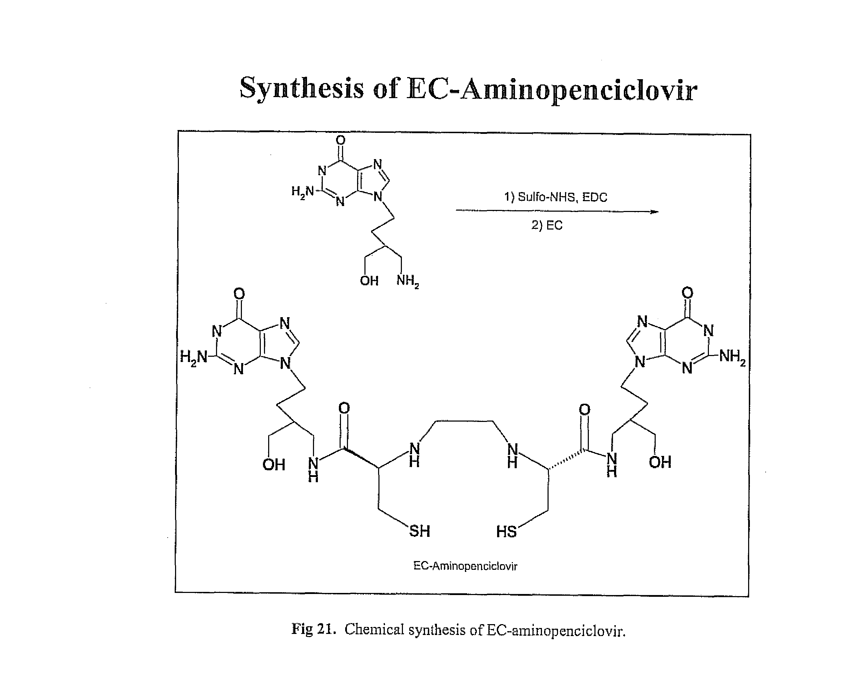

Like Penciclovir, aminopenciclovirl is a guanine analogue. It is used for targeting disease cell cycle targets. Synthesis of EC-Aminopenciclovir was accomplished in a three-step manner (shown in FIGS. 20-21). The starting material, 3-N-trityl-9-(4-tosyl-3-O-tritylmethylbutyl)guanine (penciclovir analogue), was prepared according to a published method (Allaudin 2001). Penciclovir analogue (500mg, 0.52mmol) was dissolved in N,N-dimethylformamide (DMF, 15ml). To this solution, sodium azide (160 mg, 2.5mmol) was added. The reaction was heated at 100°C for overnight. The solvent was mixed with water and extracted with ethylacetate. The solvent was evaporated to dryness under vacuo. The azido product weighed 400mg (yield 93%). Without further purification, the azido penciclovir analogue (400 mg, 0.48 mmol) was reduced with triphenylphosphine (655 mg, 2.5 mmol) in tetrahydrofuran (THF, 15 ml). The reaction was stirred overnight. Hydrochloric acid (5N, 0.5 ml) was added and the reaction was refluxed for 5 hours. The solvent was then evaporated. The reaction mixture was washed with water and extracted with ethylacetate. The water layer was collected and pH was adjusted to 7-8 using NaHCO₃ (IN). After freeze drying, the intermediate aminopenciclovir was obtained. It weighed 120 mg (90%).

To a stirred solution of EC (50 mg, 0.2 mmol) in NaHCO₃ (IN) (2 ml), sulfo-NHS (95.5 mg, 0.44 mmol) and EDC (84.5 mg, 0.44 mmol) were added. The aminopenciclovir (obtained in the preceding steps) containing saline (350 mg, 1.38 mmol) was then added. The mixture was stirred at room temperature for 24 hours. The mixture was dialyzed for 48 hours using Spectra/POR molecular porous membrane with molecule cut-off at 500 (Spectrum Medical Industries Inc., Houston, TX). After dialysis, the product was freeze dried. The product EC-aminopenciclovir weighed 100 mg (yield 67%).

### b. In Vitro Cellular Uptake Assays

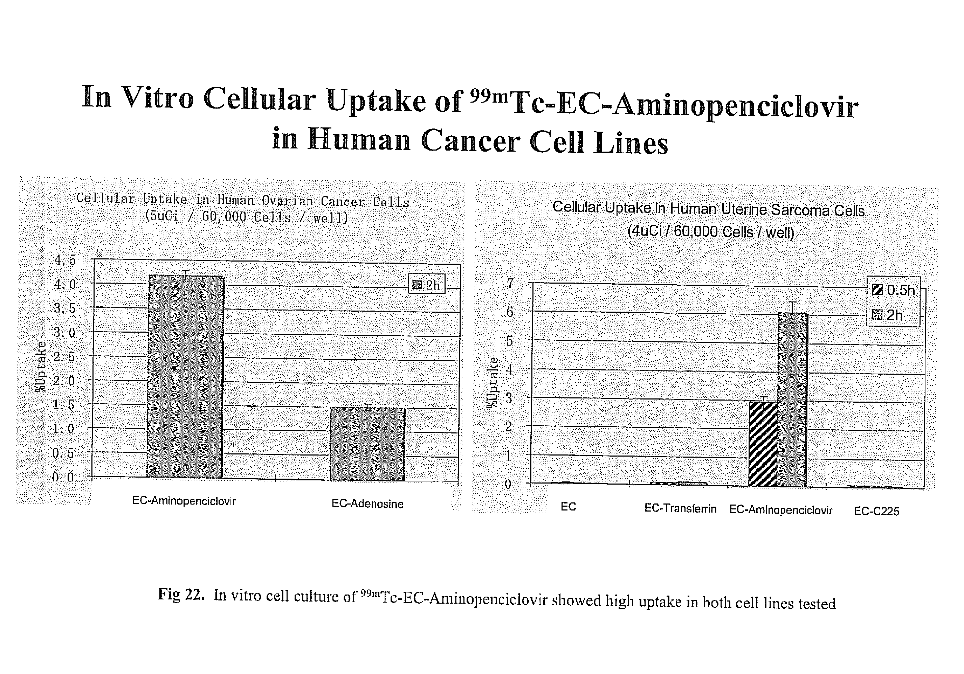

An *in vitro* cell culture using tumor cells was incubated with *^{99m}*Tc-EC-aminopenciclovir (4-6 µCi/50,000 cells/well) at 0.5-2 hrs in human ovarian and uterine cancer cells. There was a significant increased uptake compared to *^{99m}*Tc-EC (FIG. 22).

### c. Scintigraphic Imaging Studies

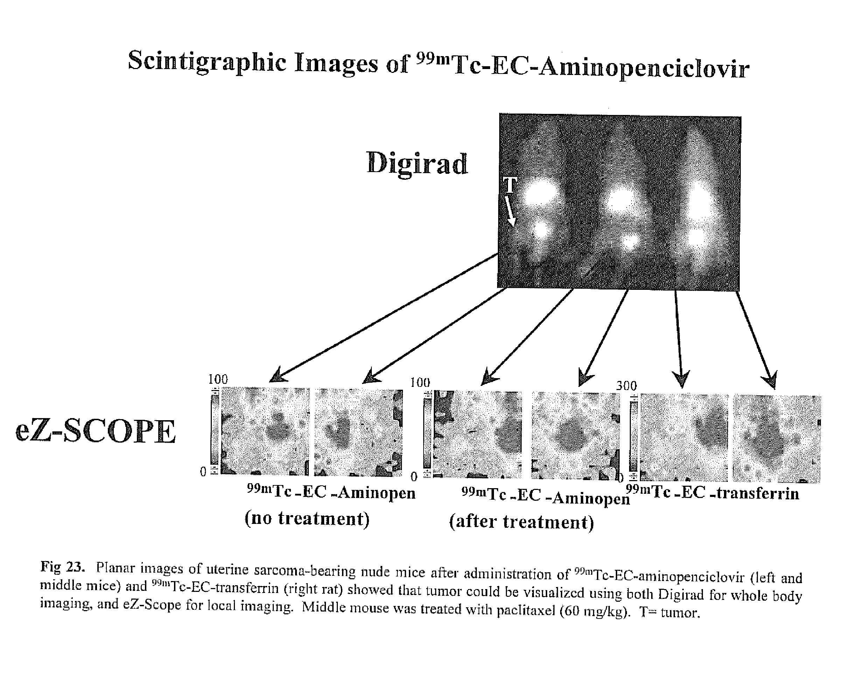

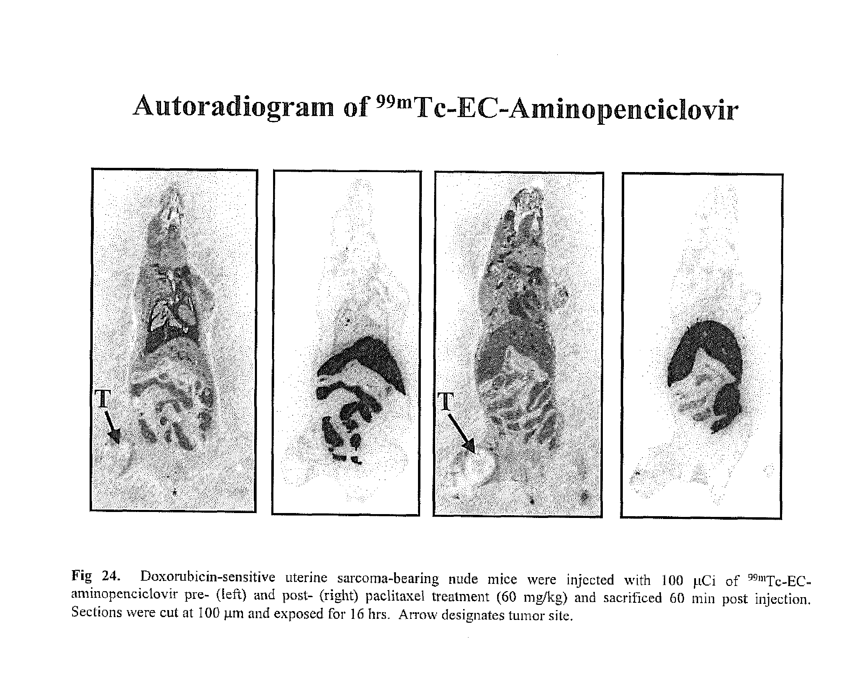

Scintigraphic imaging studies were performed in human uterine tumor-bearing mice at 0.5-4 hrs (0.1 mCi/rat, n=3, iv). Control group was administered *^{99m}*Tc-EC. Planar images confirmed that the tumors could be visualized with *^{99m}*Tc-EC-aminopenciclovir (FIG. 23). Whole-body autoradiogram was obtained by a quantitative image analyzer (Cyclone Storage Phosphor System, Packard, Meridian, CT). Following i.v. injection of 0.1m Ci of *^{99m}*Tc-EC-aminopenciclovir, the animal was killed at 1 h and the body was fixed in carboxymethyl cellulose (4%). The frozen body was mounted onto a cryostat (LKB 2250 cryomicrotome) and cut into 100 µm coronal sections. Each section was thawed and mounted on a slide. The slide was then placed in contact with multipurpose phosphor storage screen (MP, 7001480) and exposed for 15 hrs. Autoradiograms performed at 1 hr after injection of *^{99m}*Tc-EC-aminopenciclovir demonstrated the tumor activity (FIG. 24).

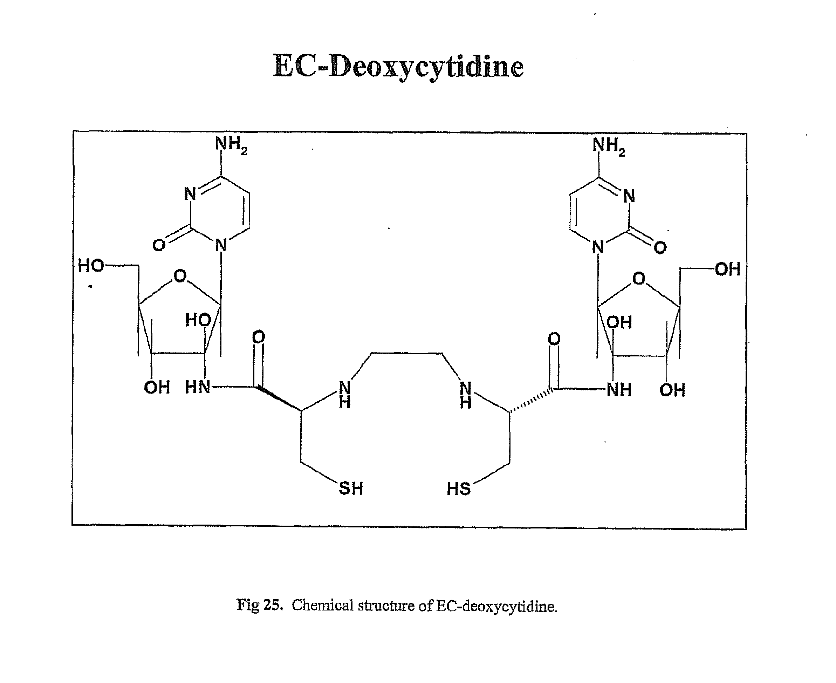

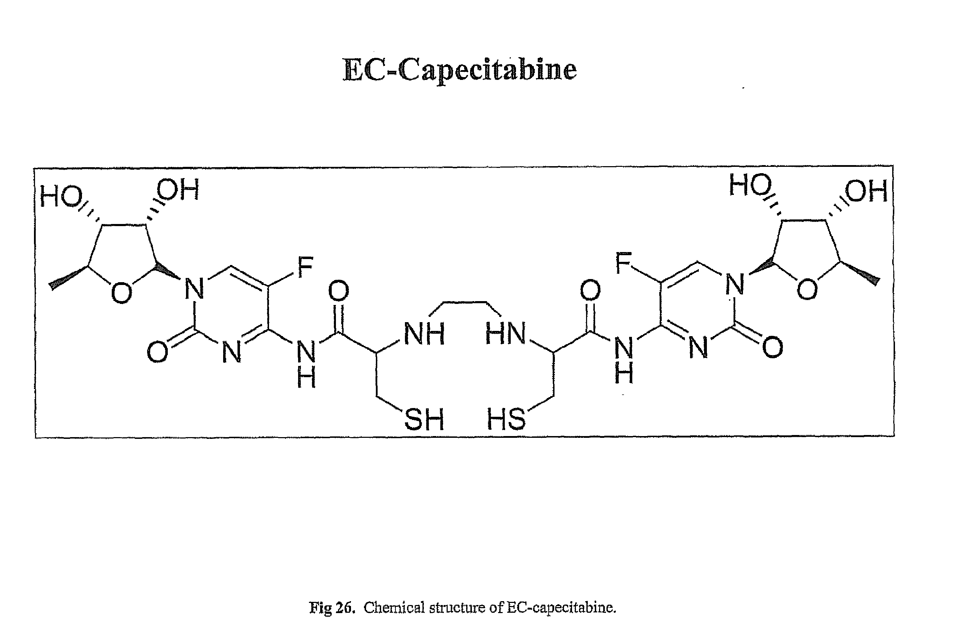

Other small molecules that can be applied using EC technology under this example, include EC-deoxyciytidine, EC-capcitabine (cytidine analogues). The structures are shown in FIGS. 25-26.

### EXAMPLE 5: TARGETING CHROMATIN WITH EC-ADENOSINE

### a. Synthesis of EC-Adenosine

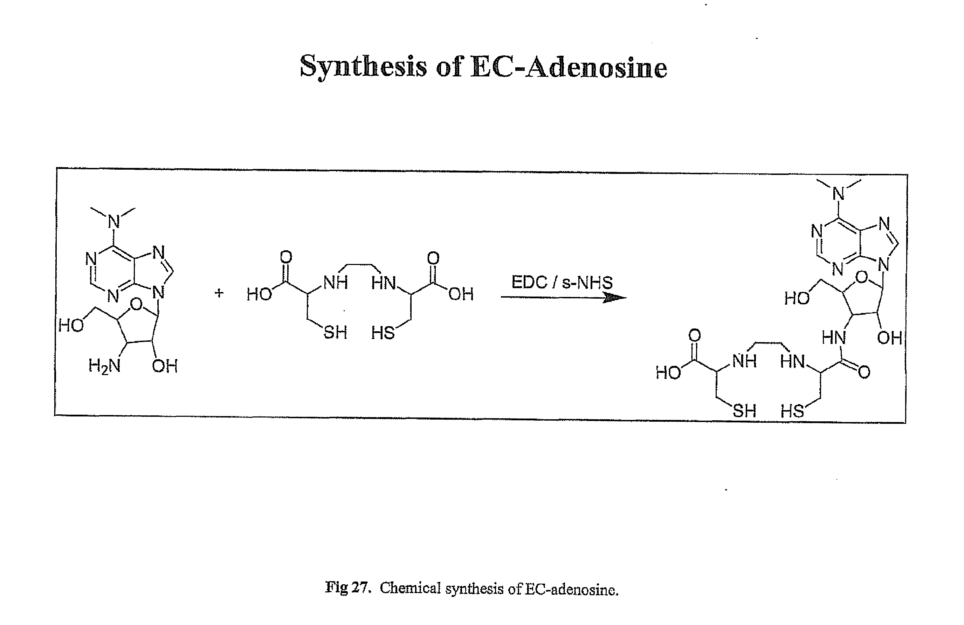

Chromatin is a disease cell cycle target that can be targeted with EC-adenosin, a compound of the present invention. To a stirred solution of EC (45.6 mg, 0.17 mmol) in NaHCO₃ (IN) (0.7 ml), sulfo-NHS (80.3 mg, 0.37 mmol) and EDC (71.0 mg, 0.37 mmol) were added. The starting material, N,N-dimethyl-3'-amino adenosine (amino adenosine analogue) (50mg, 0.17mmol) was then added. The mixture was stirred at room temperature for 24 hours. The mixture was dialyzed for 48 hours using Spectra/POR molecular porous membrane with molecule cut-off at 500 (Spectrum Medical Industries Inc., Houston, TX). After dialysis, the product was freeze dried. The product weighed 65.2 mg (yield 69.4%). Synthesis of EC-Adenosine (EC-ADN) is shown in FIG. 27.

### b. In Vitro Cellular Uptake Assays

An *in vitro* cell culture using tumor cells was incubated with *^{99m}*Tc-EC-adenosine (4-6 µCi/50,000 cells/well) at 0.5-2 hrs in human ovarian cancer cells. There was a significant increased uptake compared to *^{99m}*Tc-EC (FIG. 22).

### c. Autoradiogram Studies

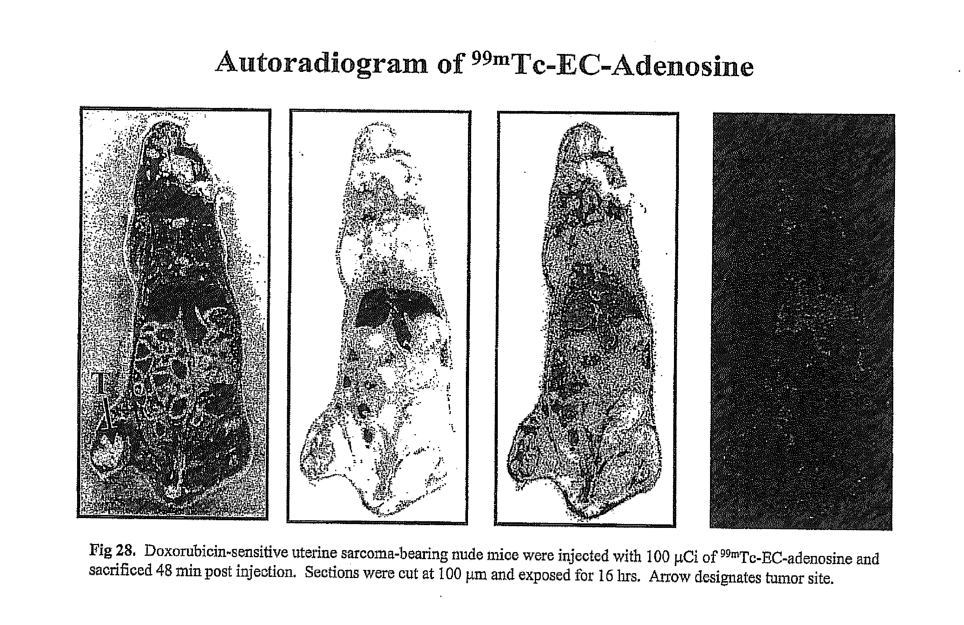

Whole-body autoradiogram was obtained by a quantitative image analyzer (Cyclone Storage Phosphor System, Packard, Meridian, CT). Following i.v. injection of 0.1 mCi of ⁹⁹M Tc-EC-adenosine, animal was killed at 1 hr and the body was fixed in carboxymethyl cellulose (4%). The frozen body was mounted onto a cryostat (LKB 2250 cryomicrotome) and cut into 100 µm coronal sections. Each section was thawed and mounted on a slide. The slide was then placed in contact with multipurpose phosphor storage screen (MP, 7001480) and exposed for 15 hrs. Autoradiograms performed at 1 hr after injection of *^{99m}*Tc-EC-adenosine demonstrated the tumor activity (FIG. 28).

### EXAMPLE 6: TARGETING GnRH RECEPTORS WITH EC-LHRH

### a. Synthesis of EC-LHRH

The GnRH receptor is a disease tissue receptor that can be targeted with EC-LHRH, a compound of the current invention. To a stirred solution of EC (4.6 mg, 0.017 mmol) in NaHCO₃ (IN) (0.5 ml), sulfo-NHS (3.72 mg, 0.017 mmol) and EDC (3.3 mg, 0.017 mmol) were added. The starting material, luteinizing hormone releasing hormone (LHRH human, Sigma Chemical Company, St. Louis, MO) (50mg, 0.042 mmol) was then added. The mixture was stirred at room temperature for 24 hours. The mixture was dialyzed for 48 hours using Spectra/POR molecular porous membrane with molecule cut-off at 1,000 (Spectrum Medical Industries Inc., Houston, TX). After dialysis, the product was freeze dried. The product weighed 51 mg (yield 93.4%).

### b. In Vitro Cellular Uptake Assays

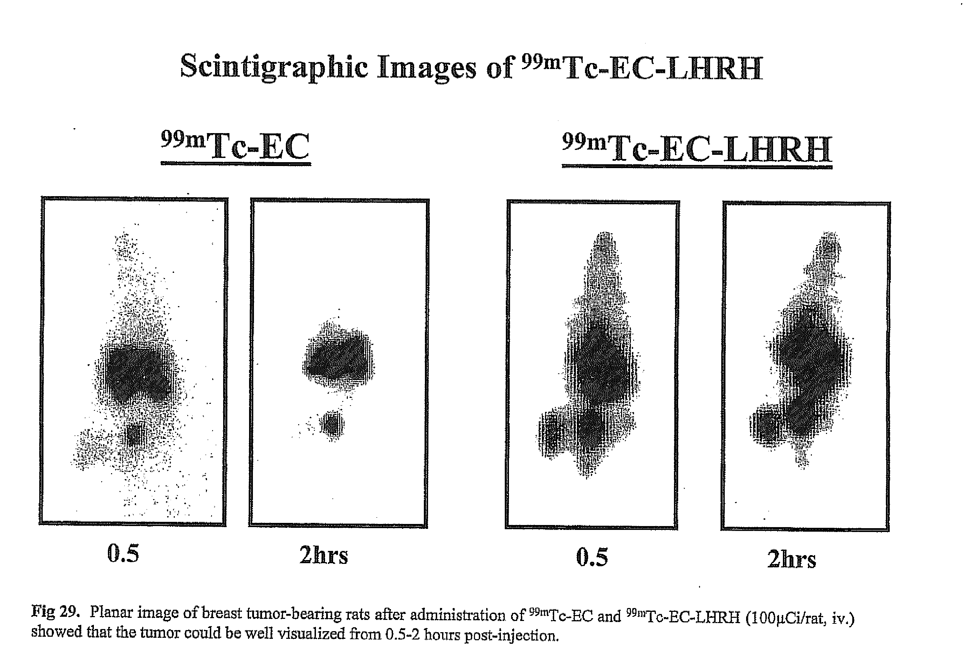

An *in vitro* cell culture using tumor cells was incubated with *^{99m}*Tc-EC-LHRH (4-6 µCi/100,000 cells/well) at 0.5-2 hrs in human prostate cancer cells. Two types cell lines were used. They are either sensitive to androgen (LNCap) or unresponsive to androgen (PC-3) therapy. There was a significant increased uptake compared to *^{99m}*Tc-EC (FIG. 29).

### c. Scintigraphic Imaging Studies

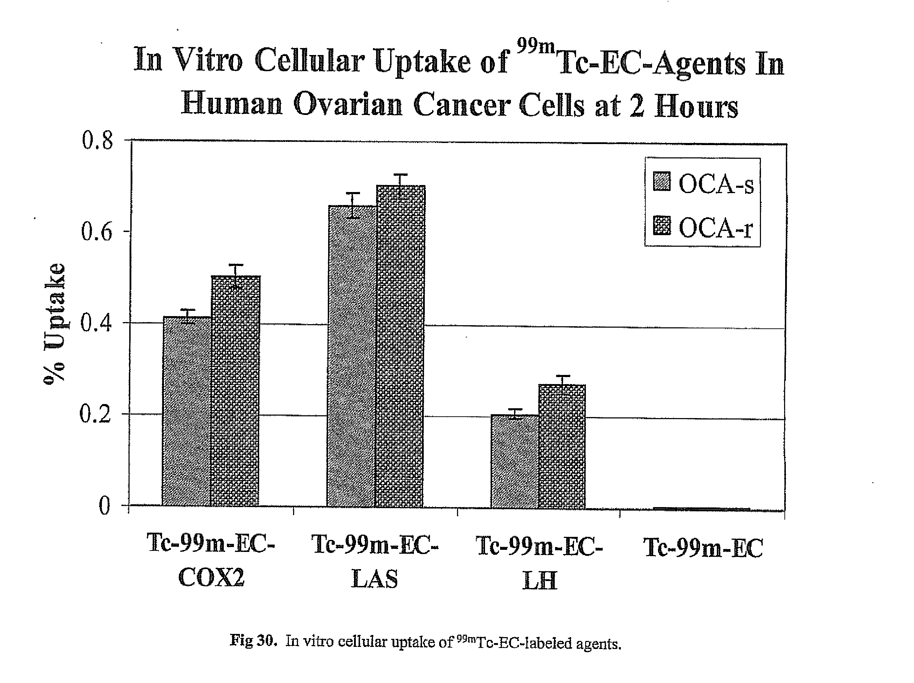

Scintigraphic imaging studies was performed in mammary tumor-bearing rats at 0.5-4 hrs (0.1 mCi/rat, n=3, iv). Control group was administered *^{99m}*Tc-EC. Planar images confirmed that the tumors could be visualized with *^{99m}*Tc-EC-LHRH (FIG. 30).

### EXAMPLE 7: TARGETING LUTEINIZING HORMONE RECEPTORS WITH EC-LH ANTIBODY

### a. Synthesis of EC-LH Antibody

The luteinizing hormone receptors are a disease tissue receptor that can be targeted with EC-LH antibody, a compound of the current invention. To a stirred solution of EC (0.51mg, 1.90µmol) in NaHCO₃ (IN) (0.1 ml), sulfo-NHS (0.41mg, 1.9µmol) and EDC (0.36mg, 1.9µmol) were added. The starting material, luteinizing hormone antibody (Sigma Chemical Company, St. Louis, MO) (5.1mg) was then added. The mixture was stirred at room temperature for 18 hours. The mixture was dialyzed for 48 hours using Spectra/POR molecular porous membrane with molecule cut-off at 10,000 (Spectrum Medical Industries Inc., Houston, TX). After dialysis, the product was freeze dried. The product weighed 5.1 mg (yield 97%).

### b. In vitro Cellular Uptake Assays and Tissue Distribution Studies

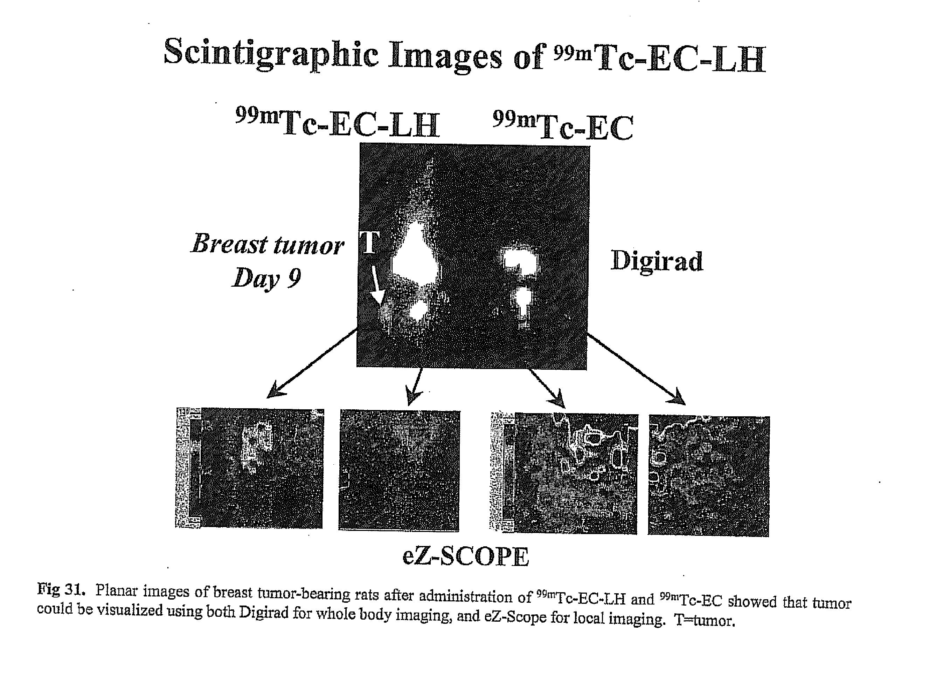

An *in vitro* cell culture using tumor cells was incubated with *^{99m}*Tc-EC-LH antibody (4 µCi/50,000 cells/well) at 0.5-2 hrs in RBA CRL-1747 (breast cancer cells). There was a significant increased uptake compared to *^{99m}*Tc-EC (FIG. 31).

Biodistribution was assessed in breast tumor-bearing rats (RBA CRL-1747, n=3/time interval, iv). Studies were performed 14 to 17 days after implantation when tumors reached approximately 1cm in diameter. Following administration of the radiotracer, rats were sacrificed at 0.5-4 hrs. The selected tissues were excised, weighed and counted for radioactivity by using a gamma counter (Packard Instruments, Downers Grove, IL). The biodistribution of tracer in each sample was calculated as percentage of the injected dose per gram of tissue wet weight (%ID/g). Biodistribution of *^{99m}*Tc-EC-LH antibody in tumor-bearing rats showed increased tumor-to-tissue count density ratios as a function of time compared to *^{99m}*Tc-EC (Tables 2 and 3).

**TABLE 4: Biodistribution of ^{99m}Tc-EC-LH in Breast Tumor-Bearing Rats % of injected dose per gram of tissue weight (n=3/time, interval,iv)**

| **30 min** | | | **2h** | | **4h** | |
|---|---|---|---|---|---|---|
| BLOOD | 5.368 | ±0.112 | 3.517 | ±0.058 | 2.624 | ±0.014 |
| HEART | 0.978 | ±0.114 | 0.660 | ±0.036 | 0.524 | ±0.001 |
| LUNG | 2.060 | ±0.185 | 1.343 | ±0.042 | 1.057 | ±0.018 |
| LIVER | 2.445 | ±0.089 | 1.833 | ±0.093 | 1.518 | ±0.091 |
| SPLEEN | 1.506 | ±0.117 | 1.260 | ±0.092 | 0.929 | ±0.050 |
| KIDNEY | 9.450 | ±0.313 | 12.596 | ±0.179 | 12.257 | ±0.482 |
| INTESTINE | 0.656 | ±0.073 | 0.526 | ±0.031 | 0.483 | ±0.017 |
| UTERUS | 0.953 | ±0.044 | 0.965 | ±0.092 | 0.559 | ±0.071 |
| MUSCLE | 0.161 | ±0.012 | 0.138 | ±0.015 | 0.085 | ±0.010 |
| TUMOR | 0.776 | ±0.065 | 0.701 | ±0.029 | 0.699 | ±0.034 |
| THYROID | 0.852 | ±0.081 | 0.693 | ±0.169 | 0.762 | ±0.075 |
| STOMACH | 0.593 | ±0.103 | 0.491 | ±0.101 | 0.349 | ±0.007 |
| BONE | 0.619 | ±0.014 | 0.547 | ±0.094 | 0.359 | ±0.033 |
| TUMOR/MUSCLE | 4.898 | ±0.599 | 5.143 | ±0.363 | 8.569 | ±1.359 |
| TUMOR/BLOOD | 0.145 | ±0.014 | 0.200 | ±0.01 | 0.266 | ±0.014 |
| UTERUS/BLOOD | 0.178 | ±0.010 | 0.275 | ±0.027 | 0.213 | ±0.026 |
| UTERUS/MUSCLE | 5.951 | ±0.200 | 7.011 | ±0.391 | 6.592 | ±0.039 |
| BONE/MUSCLE | 3.876 | ±0.213 | 4.076 | ±0.916 | 4.378 | ±0.747 |

Values shown represent the mean ±standard deviation of data from 3 animals.

### c. Scintigraphic Imaging Studies

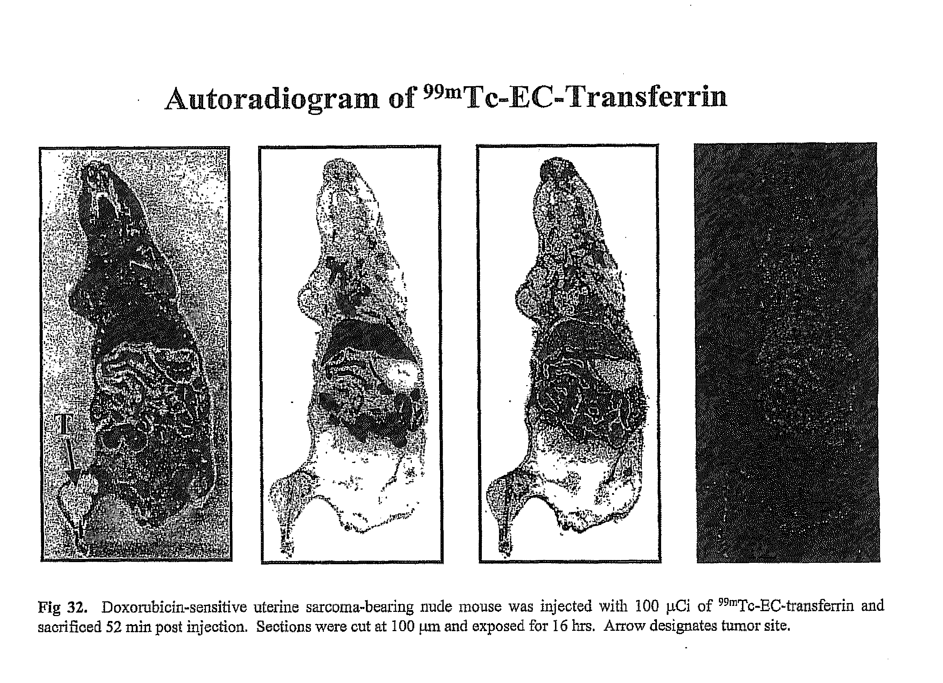

Scintigraphic imaging studies was performed in breast tumor-bearing rats at 0.5-4 hrs (0.1 mCi/rat, n=3, iv). Control group was administered *^{99m}*Tc-EC. Planar images confirmed that the tumors could be visualized with *^{99m}*Tc-EC-penciclovir (FIG. 32).

### EXAMPLE 8: TARGETING TRANSFERRIN RECEPTORS WITH EC-TRANSFERRIN

### a. Radiosynthesis of ^{99m}Tc-EC-Transferrin

Transferrin receptors are a disease tissue receptor that can be targeted with EC-transferrin, a compound of the current invention. Transferrin (100 mg) was stirred with EC (15 mg, 0.056 mmol in 1.0 ml of IN NaHCO₃), sulfo-NHS (11.6 mg, 0.056 mmol) and EDC (10.7 mg, 0.056 mmol). After dialysis (cut off at MW 10,000) , 110 mg of EC-transferrin (96%) was obtained. 100 mCi of Na*^{99m}*TcO₄ was added into a vial containing 5 mg EC-C225 and 100 µg SnCl₂ and the product was purified with a G-25 column and eluted with PBS, yielded 80 mCi *^{99m}*Tc-EC-transferrin.

### b. Scintigraphic Imaging and Autoradiogram Studies

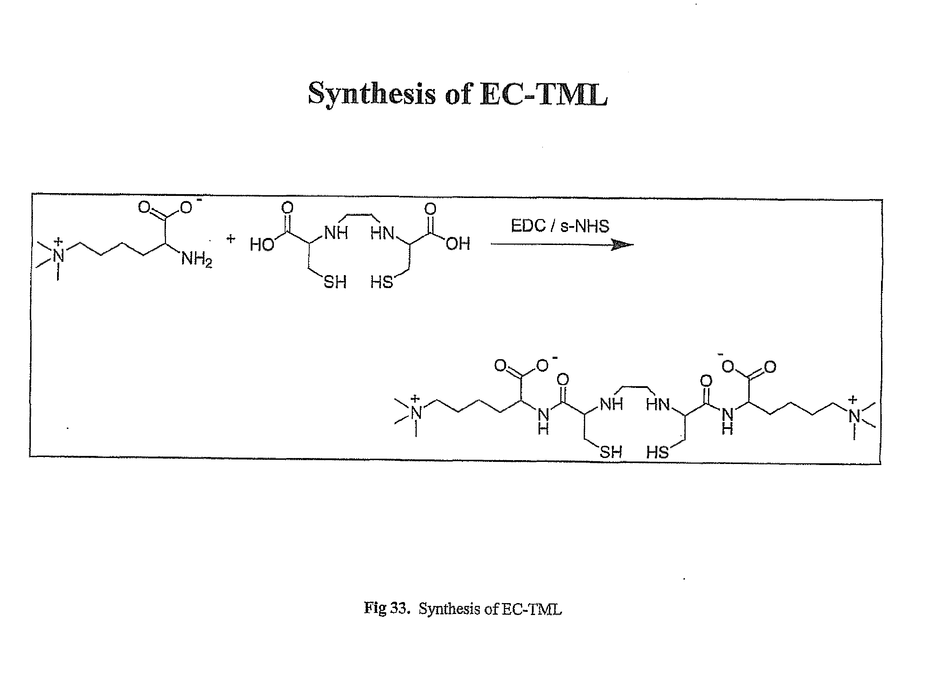

Scintigraphic imaging studies was performed in human uterine tumor-bearing mice at 0.5-4 hrs (0.1 mCi/rat, n=3, iv). Control group was administered *^{99m}*Tc-EC. Planar images confirmed that the tumors could be visualized with *^{99m}*Tc-EC-transferrin (FIG. 23). Whole-body autoradiogram was obtained by a quantitative image analyzer (Cyclone Storage Phosphor System, Packard, Meridian, CT). Following i.v. injection of 0.1 mCi of *^{99m}*Tc-EC-transferrin, animal was killed at 1 hr and the body was fixed in carboxymethyl cellulose (4%). The frozen body was mounted onto a cryostat (LKB 2250 cryomicrotome) and cut into 100 µm coronal sections. Each section was thawed and mounted on a slide. The slide was then placed in contact with multipurpose phosphor storage screen (MP, 7001480) and exposed for 15 hrs. Autoradiograms performed at 1 hr after injection of *^{99m}*Tc-EC-transferrin demonstrated the tumor activity (FIG. 33).

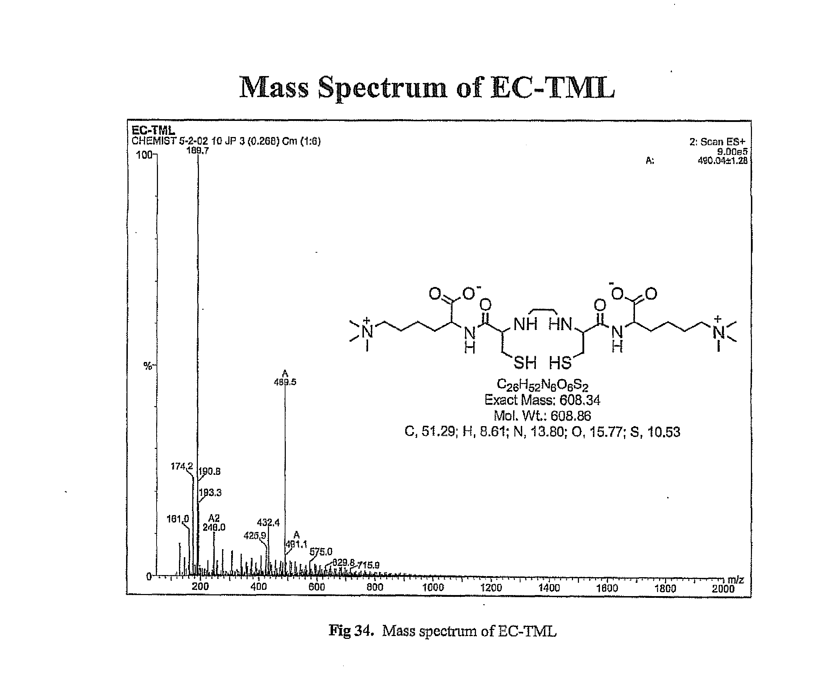

Other proteins and peptides can be applied using the EC technology, including EC-somatostatin, EC-caspase, EC-endorphin, EC-PSA, EC-p53, EC-octreotide (structure is shown in FIG. 34).

### EXAMPLE 9: TARGETING TUMOR TOPOISOMERASE WITH EC-DOXORUBICIN

### a. Synthesis of EC-Doxorubicin

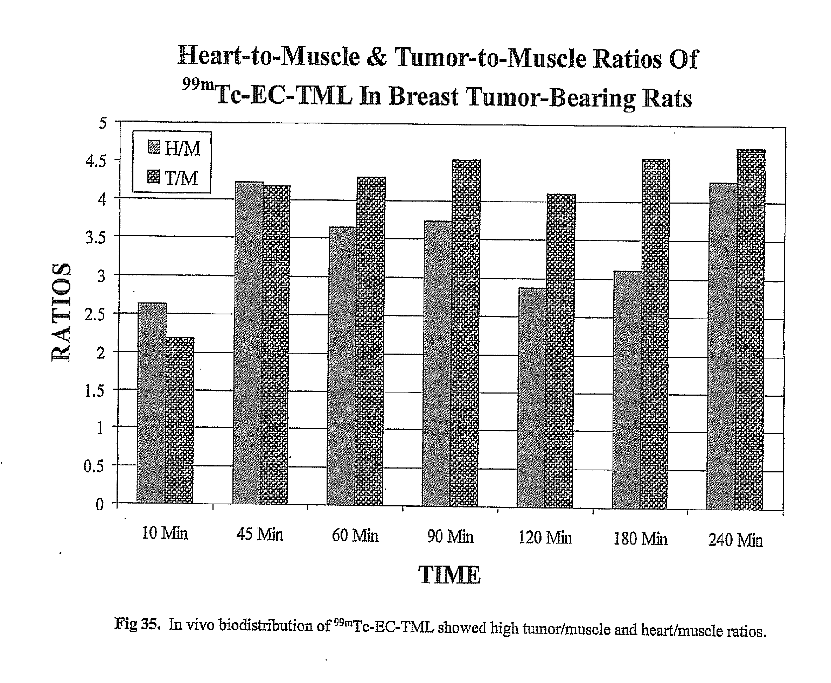

The response of tissue to theraputic drugs can be targeted with the compounds of the current invention. Tumor topoisomerase is targeted with EC-doxorubicin, a compound of the current invention. To a stirred solution of EC (55.1 mg, 0.21 mmol) in NaHCO₃ (IN) (2 ml), sulfo-NHS (44.6 mg, 0.21 mmol) and EDC (39.4 mg, 0.21 mmol) were added. The starting material, doxorubicin (119.2 mg, 0.21mmol) was then added. The mixture was stirred at room temperature for 24 hours. The mixture was dialyzed for 48 hours using Spectra/POR molecular porous membrane with molecule cut-off at 500 (Spectrum Medical Industries Inc., Houston, TX). After dialysis, the product was freeze dried. The product weighed 135 mg (yield 78%). Synthesis of EC-doxorubicin (EC-Doxo) is shown in FIG. 35.

### b. In Vitro Cellular Uptake Assays

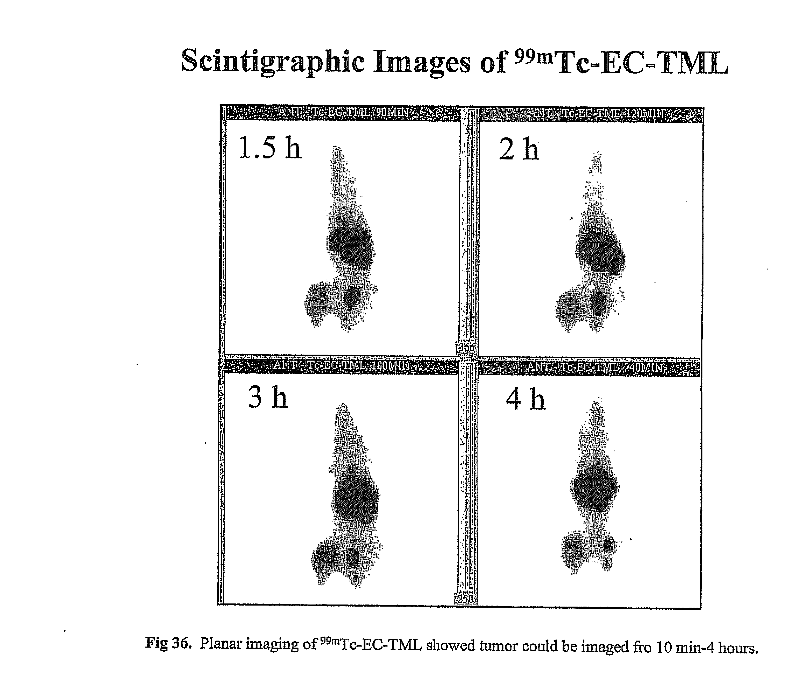

An *in vitro* cell culture using tumor cells was incubated with *^{99m}*Tc-EC-doxorubicin (4-6 µCi/50,000 cells/well) at 0.5-2 hrs in human breast cancer cells sensitive (MDA 231, low HER2) and resistant to doxorubicin (MDA 453, high HER2). There was more uptake in doxorubicin-sensitive cells than doxorubicin-resisitant cells (FIG. 36).

Other small molecules that can be applied using EC technology under this example, including EC-paclitaxel, EC-topotecan, EC-flutamide, EC-antisense, EC-tamoxifen, EC-mitoxantrone, EC-mitomycin, EC-vancomycin, EC-bleomycin.

### EXAMPLE 10: TARGETING LIPID METABOLISM WITH EC-CARNITINE

### a. Synthesis of EC-Carnitine (EC-TML)

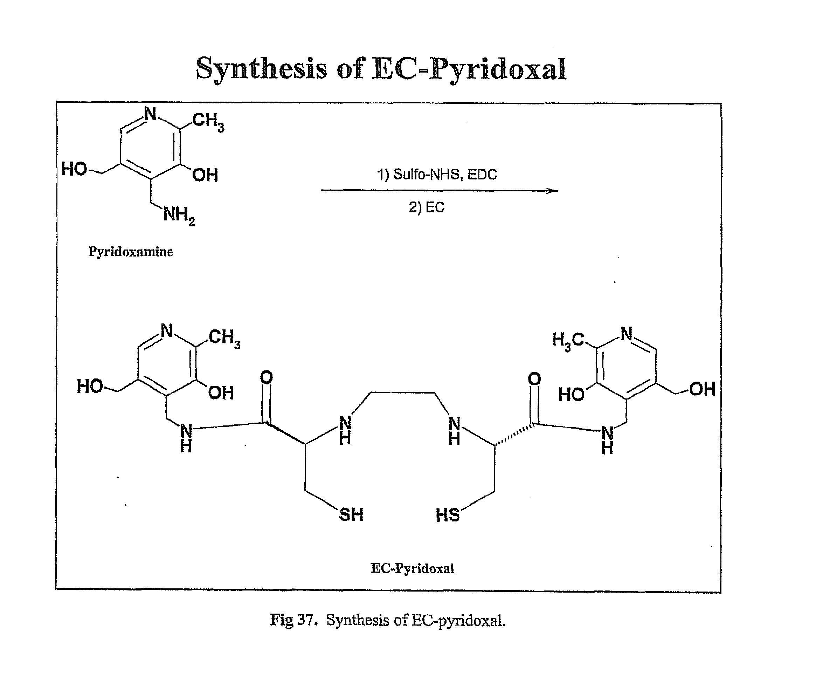

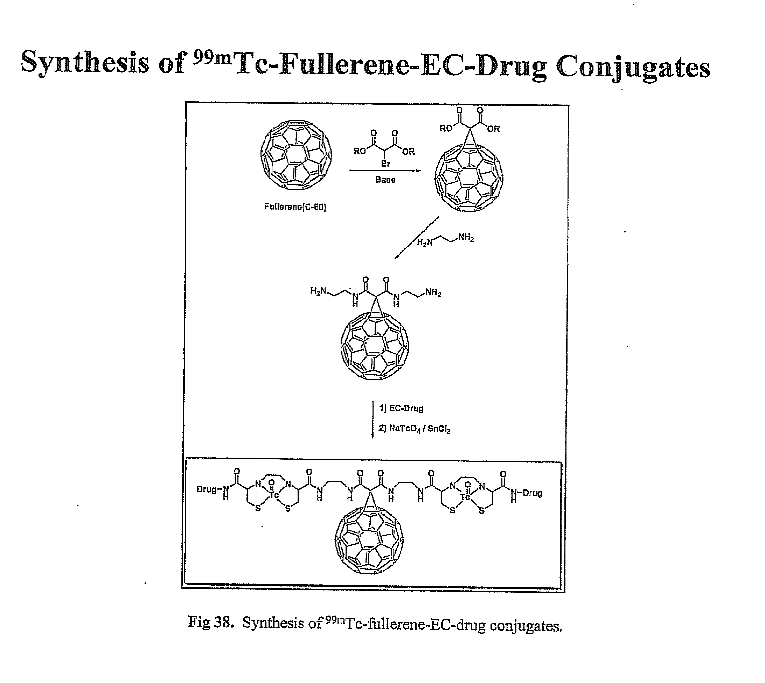

Carnitine, 2-hydroxy-3-trimethylammonium butyrate, is important for the oxidation of fatty acid and is an example of targeting disease signal transduction pathways. 6-Trimethylammonium lysine (TML) is an analogue of carnitine. EC was conjugated to amino group of TML. To a stirred solution of EC (34.9 mg, 0.13 mmol) in NaOH (IN) (0.5 ml), sulfo-NHS (62 mg, 0.29 mmol) and EDC (54.8mg, 0.29 mmol) were added. The starting material, TML (50 mg, 0.13mmol) was then added. The mixture was stirred at room temperature for 24 hours. The mixture was dialyzed for 48 hours using Spectra/POR molecular porous membrane with molecule cut-off at 500 (Spectrum Medical Industries Inc., Houston, TX). After dialysis, the product was freeze dried. The product weighed 74.2 mg (yield 93.8%). Synthesis of EC-carnitine (EC-TML) is shown in FIG. 37. Mass spectra of EC-TML is shown in FIG. 38.

### b. In Vitro Cellular Uptake Assays

An *in vitro* cell culture using tumor cells was incubated with *^{99m}*Tc-EC-carnitine (4-6 µCi/50,000 cells/well) at 0.5-2 hrs in breast cancer cells. There was more uptake in *^{99m}Tc-*EC-carnitine than *^{99m}*Tc-EC (FIG. 16).

### c. Scintigraphic Imaging Studies

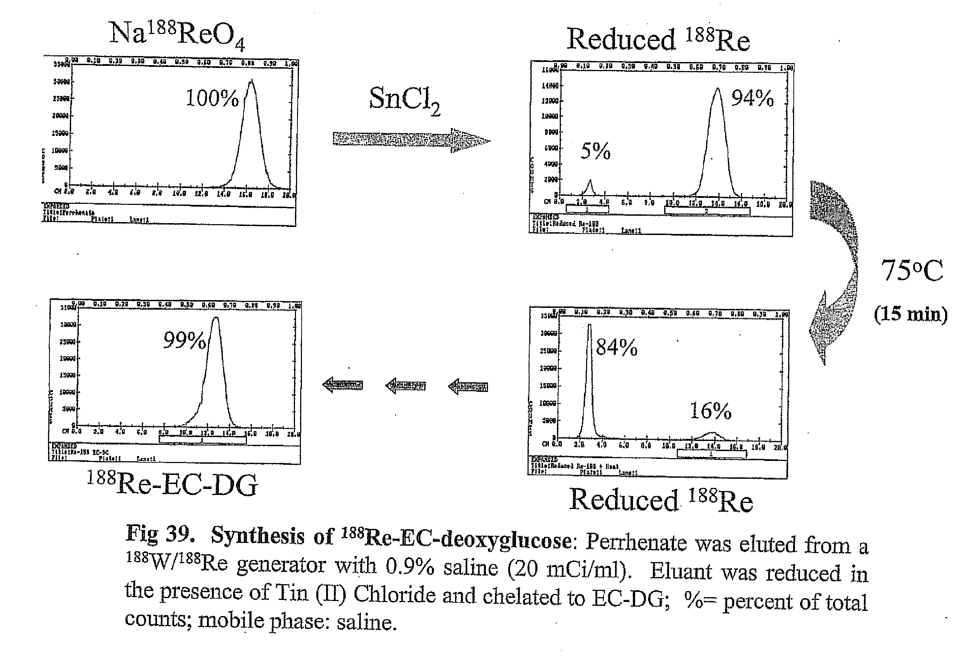

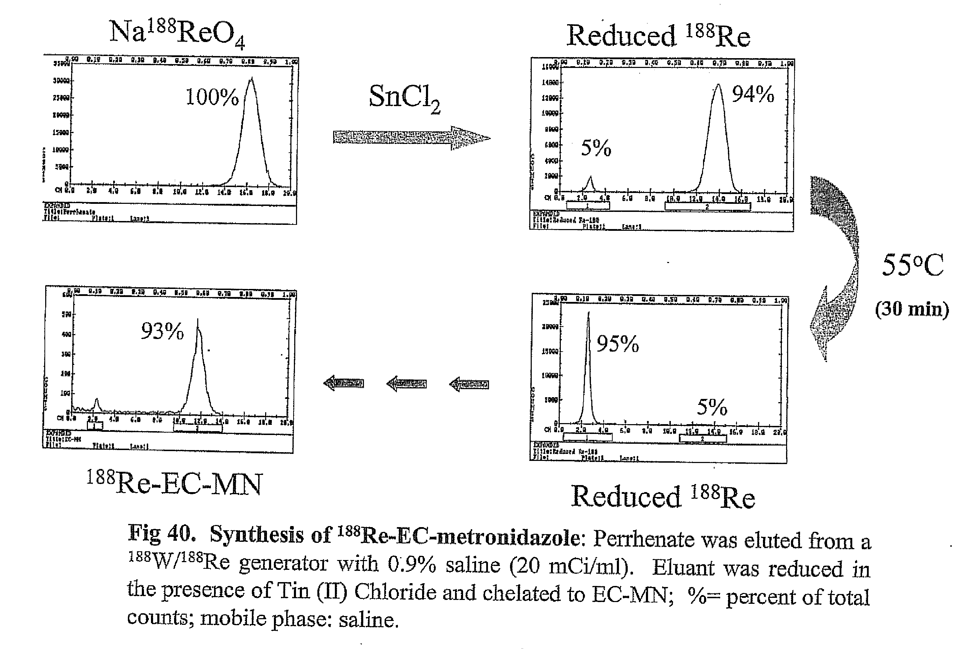

Scintigraphic imaging studies was performed in breast tumor-bearing rats at 0.1-4 hrs (0.1 mCi/rat, n=3, iv). Control group was administered *^{99m}*Tc-EC. High count density ratios were observed with tumor/muscle and heart/muscle (FIG. 39). Planar images confirmed that the tumors could be visualized with *^{99m}*Tc-EC-TML (FIG. 40).

### EXAMPLE 11: TARGETING GLUCOSAMINE AND GLUCOSE METABOLISM WITH EC-DEOXYGLUCOSE

### a. Synthesis of EC-deoxyglucose (EC-DG)

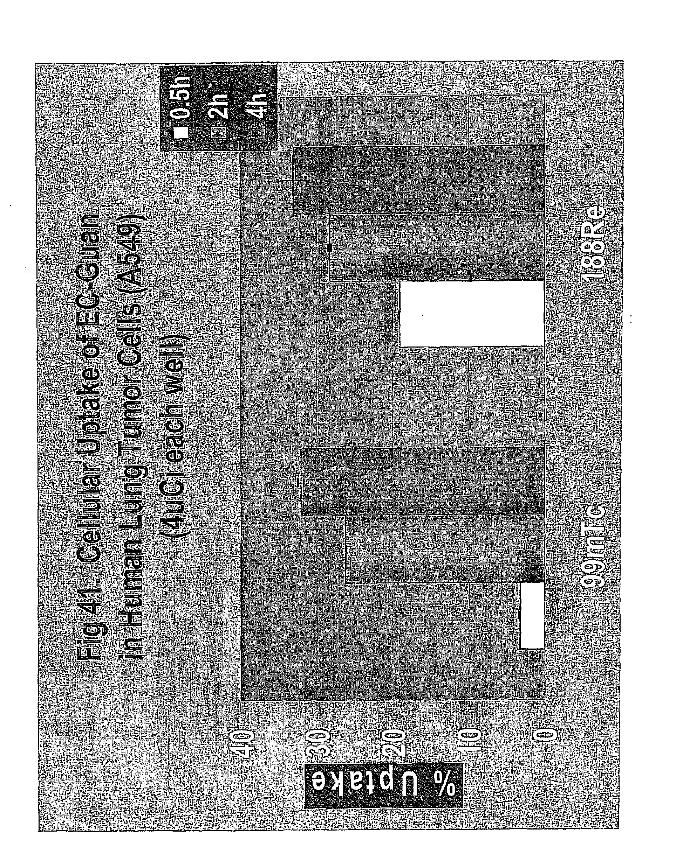

A signal transduction pathway, glucosamine metabolism was targeted with EC-deoxyglucose, a compound of the current invention. Sodium hydroxide (1N, 1ml) was added to a stirred solution of EC (110 mg, 0.41 mmol) in water (5 ml). To this colorless solution, sulfo-NHS (241.6 mg, 1.12 mmol) and EDC (218.8 mg, 1.15 mmol) were added. D-Glucosamine hydrochloride salt (356.8 mg, 1.65 mmol) was then added. The mixture was stirred at room temperature for 24 hours and pH was adjusted to 6.4-7.0. The mixture was dialyzed for 48 hours using Spectra/POR molecular porous membrane with cut-off at 500 (Spectrum Medical Industries Inc., Houston, TX). After dialysis, the product was frozen dried using lyophilizer (Labconco, Kansas City, MO). The product weighed 291 mg (yield 60%). ¹H-NMR (D₂O δ 2.60-2.90 (m, 4H and -CH₂-SH of EC), 2.95 (t, 2H, glucosamine 5-CH-CH₂OH) 3.20 (d, 4H, glucosamine 6-CH₂OH) 3.30-3.95(m, 6H glucosamine1,3,4-CH and 4H CH₂-SH of EC) 3.30-3.66 (m, 4H, CH₂-CH₂- of EC), 4.15-4.30 (t, 2H, NH-CH-CO of EC), 4.60 (d, 2H, glucosamine 2-CH -NH₂). FAB MS m/z 591 (M+, 20). Structure is shown in FIG. 41.

### b. Tissue Distribution Studies of ^{99m}Tc-EC-DG

Female athymic nude mice (NCr-nu/nu, NCI, Bethasda, MD) were inoculated with human lung cancer cells (A549 tumor cell line, 3 x 106 cells/mouse, intramuscular) by one author in the mid-dorsal region. After the tumor reached 6 mm, separate biodistribution studies using *^{99m}*Tc-EC-DG and 18F-FDG were conducted. Each received *^{99m}*Tc-EC-DG or 18F-FDG intravenously (n=3/time point). The injection activity was 1-3 µCi/mouse. The injected mass of *^{99m}*Tc-EC-DG was 0.2 mg/rodent. Following administration of the radiotracers, the rodents were sacrificed and the selected tissues were excised, weighed and counted for radioactivity. *^{99m}*Tc-EC-DG had higher tumor/muscle and tumor/brain ratios as a function of time, while 18F-FDG had higher tumor/blood ratios (Tables 4 and 5).

**TABLE 5: Biodistribution of ¹⁸FDG in Lung Tumor-Bearing Mice (% of injected dose per gram of tissue weight)**

| | **30min** | **2h** | **4h** |
|---|---|---|---|
| Blood | 0.793±0.067 | 0.236±0.009 | 0.203±0.062 |
| Lung | 2.490±0.209 | 2.222±0.137 | 2.280±0.182 |
| Liver | 1.051±0.057 | 0.586±0.040 | 0.785±0.039 |
| Stomach | 5.046±0.461 | 4.374±0.864 | 2.278±0.455 |
| Spleen | 1.824±0.196 | 1.903±0.144 | 1.591±0.161 |
| Kidney | 1.137±0.117 | 0.553±0.104 | 0.568±0.027 |
| Thyroid | 4.490±0.526 | 4.617±0.400 | 4.424±0.442 |
| Muscle | 4.876±0.621 | 5.409±0.611 | 4.743±0.610 |
| Intestine | 2.322±0.542 | 2.764±0.496 | 1.562±0.342 |
| Tumor | 2.226±0.150 | 1.699±0.172 | 1.606±0.182 |
| Brain | 6.557±0.390 | 3.113±0.132 | 2.065±0.080 |
| Heart | 11.94±2.571 | 20.33±7.675 | 19.35±8.286 |
| Tumor/Blood | 2.821±0.144 | 7.261±1.007 | 8.932±1.973 |
| Tumor/Muscle | 0.463±0.026 | 0.319±0.031 | 0.346±0.048 |
| Tumor/Lung | 0.912±0.119 | 0.775±0.113 | 0.717±0.106 |

Values shown represent the mean±standard deviation of data from 3 animals.

**TABLE 6: Biodistribution of ^{99m}Tc-EC-DG in Lung Tumor-Bearing Mice (% of injected dose per gram of tissue weight)**

| | **30 min** | **2 h** | **4 h** |
|---|---|---|---|
| Blood | 1.607±0.389 | 0.977±0.267 | 0.787±0.152 |
| Lung | 1.048±0.259 | 0.721±0.210 | 0.606±0.128 |
| Liver | 5.674±2.089 | 5.807±1.708 | 6.656±1.786 |
| Stomach | 0.540±0.113 | 0.439±0.138 | 0.541±0.119 |
| Spleen | 3.240±1.709 | 4.205±1.374 | 5.933±3.194 |
| Kidney | 6.726±1.842 | 5.687±1.540 | 4.318±0.890 |
| Thyroid | 0.929±0.212 | 0.665±0.207 | 0.692±0.119 |
| Muscle | 0.264±0.072 | 0.148±0.039 | 0.147±0.022 |
| Intestine | 0.510±0.093 | 0.417±0.110 | 0.374±0.073 |
| Tumor | 0.787±0.163 | 0.415±0.123 | 0.414±0.161 |
| Brain | 0.058±0.008 | 0.042±0.006 | 0.042±0.006 |
| Heart | 0.611±0.193 | 0.336±0.080 | 0.318±0.071 |
| Tumor/Blood | 0.499±0.022 | 0.424±0.022 | 0.502±0.120 |
| Tumor/Muscle | 3.352±0.749 | 2.754±0.120 | 2.795±0.982 |
| Tumor/Lung | 0.769±0.047 | 0.587±0.046 | 0.640±0.120 |

Values shown represent the mean ± standard deviation of data from 3 animals.

### c. Gamma Scintigraphic Imaging Studies

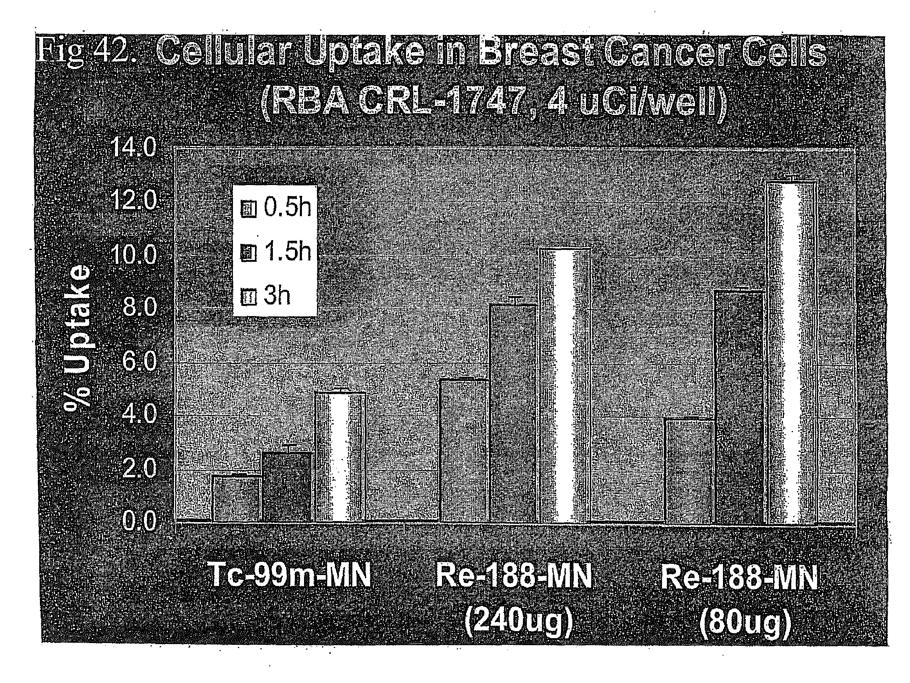

Female Fischer 344 rats (250-275g each) (Harlan, Inc., Indianapolis, IN) were inoculated with mammary tumor cells by one author from the 13762 tumor cell line (s.c. 106 cells/rat, a tumor cell line specific to Fischer rats). After 8-10 days, tumor volumes of 0.3-0.6 cm were measured. Scintigraphic images were obtained 0.5, 2 and 4 hours after i.v. injection of 300 µCi of *^{99m}*Tc-EC or *^{99m}*Tc-EC-DG (n=3/agent, total 6 rats). ROI between tumor tissue and muscle (at symmetric site) was used to determine tumor/non-tumor ratios. The smallest tumor volume that could be detected by *^{99m}*Tc-EC-DG was 3 mm. The medium-sized tumor (6 mm) showed higher uptake at each time point. Heart, kidneys, liver, and bladder were visualized (FIG. 42).

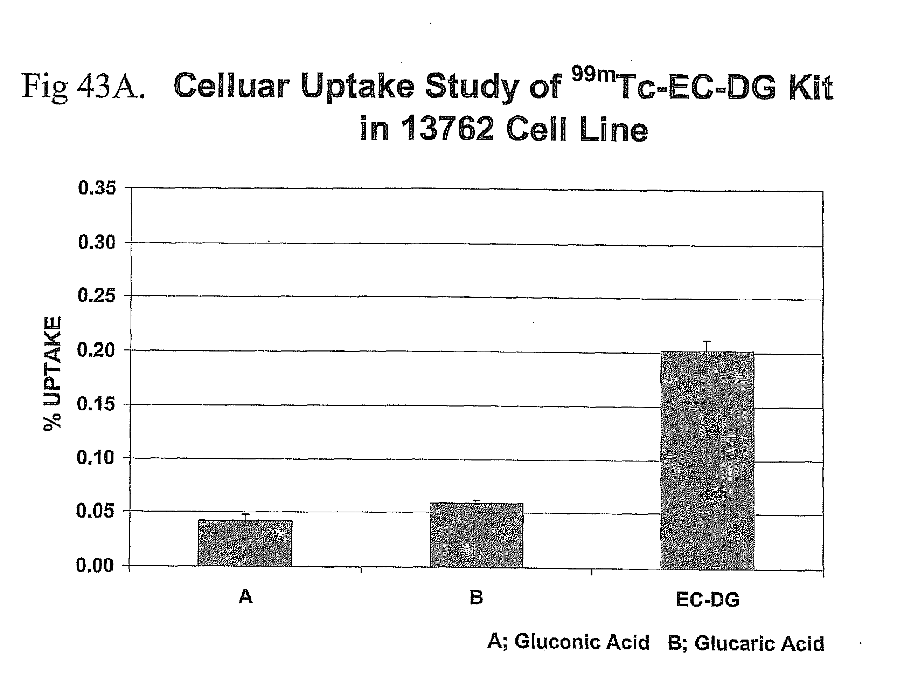

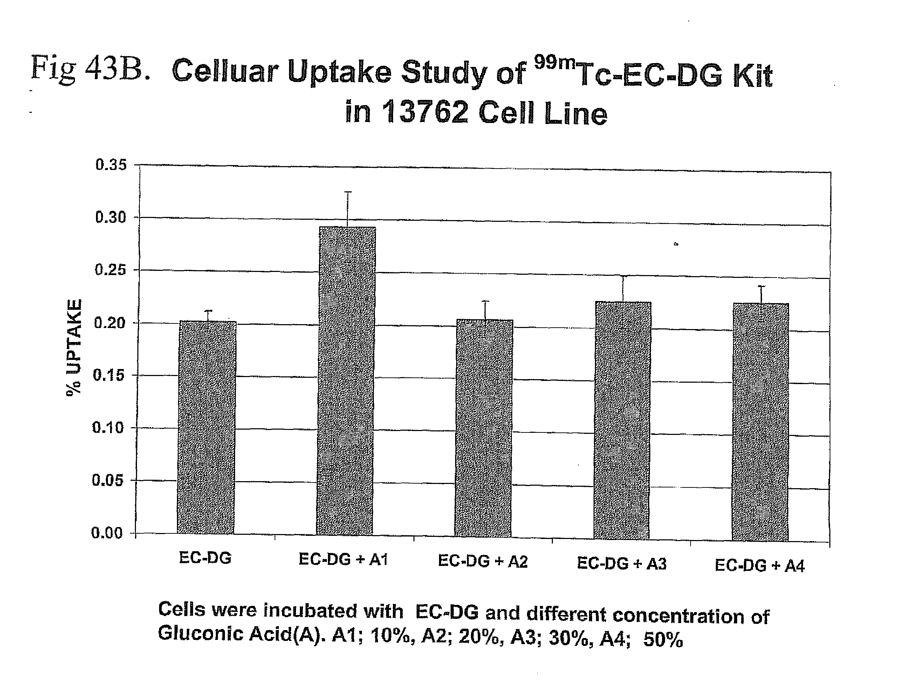

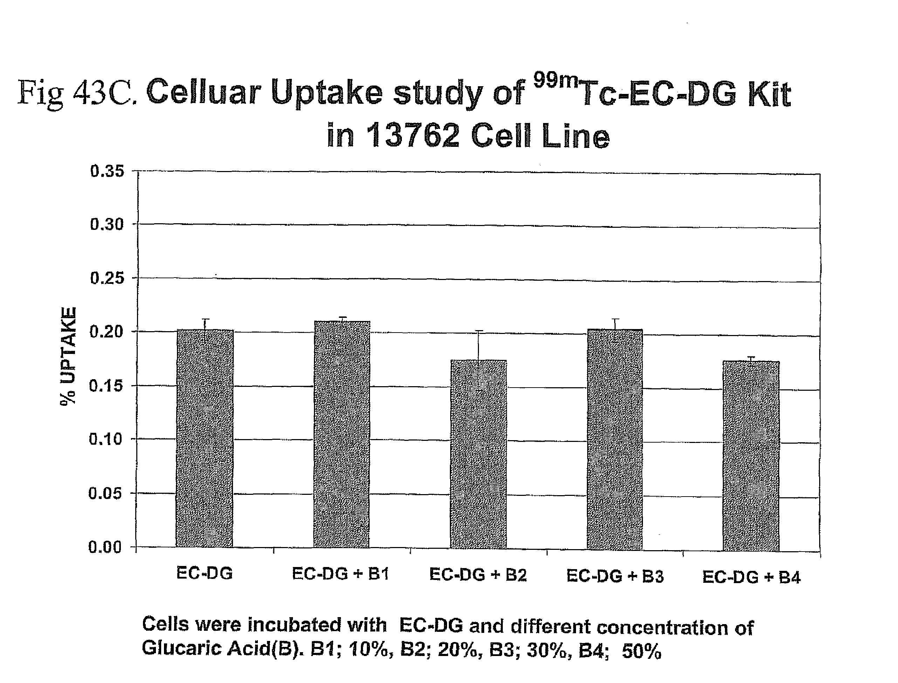

Other small molecules that can be applied using EC technology under this example, including EC-amifostine, EC-lactose, EC-pyridoxal (structure is shown in FIG. 43), EC-Fullerene (EC-carbon 60, structure is shown in FIG. 44).

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### d. Synthesis and formulation of ¹⁸⁸Re-EC-DG

To develop EC-DG in a kit form, EC-DG (10 mg) dissolved in 0.2 ml of water was freeze dried with tin chloride (2 mg in 0.2 ml of water) and gluconate (3 mg) in a 10 ml medivial and stored at room temperature prior to labeling. During labeling, the freeze dried powder was reconstituted in saline (0.5 ml) and pertechnate (10 mCi) was added. The kit was heated at 55° C for 30 min (or 75° C for 15 min). Radiochemical purity of *^{99m}*Tc-EC-DG was greater than 95% as determined by radio-TLC (saline, Rf: 0.8)(FIG. 39). A similar method could be applied to other EC-agents such as EC-metronidazole (EC-MN)(FIG. 40) and EC-penciclovir (also known as EC-Guan). To demonstrate the similarity of chemistry between Re-188 and Tc-99m, cell culture of Re-188 and Tc-99m labeled EC-Guan and EC-metronidazole was performed. There was no marked difference between uptakes (FIG. 41 and FIG. 42).

It may also be important to include an antioxidant and a transition chelator in the composition to prevent oxidation of the ethylenedicysteine. For example, the antioxidant may be vitamin C (ascorbic acid). However, other antioxidants known to those of ordinary skill in the art, such as tocopherol, pyridoxine, thiamine, or rutin, may also be used. Any transition chelator known to those of ordinary skill in the art may be used in conjunction with the present invention. Examples of transition chelators include glucoheptonate, gluconate, glucarate, citrate, and tartarate. In certain embodiments, the transition chelator is gluconate or glucarate, which do not interfere with the stability of ethylenedicysteine. *In vitro* cell culture of ^{99m}Tc-EC-deoxyglucose (EC-DG) with or without transition chelators (gluconate and glucarate) does not interfere with the stability of ^{99m}Tc-EC-DG (FIG. 43A-43C).

The following 55 embodiments are preferred embodiments of the invention and further characterize the invention:
1. A compound that comprises an N₂S₂ chelate conjugated to a targeting ligand, wherein the targeting ligand is a disease cell cycle targeting compound, a tumor angiogenesis targeting ligand, a tumor apoptosis targeting ligand, a disease receptor targeting ligand, amifostine, angiostatin, monoclonal antibody C225, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine.
2. The compound of embodiment 1, further defined as: 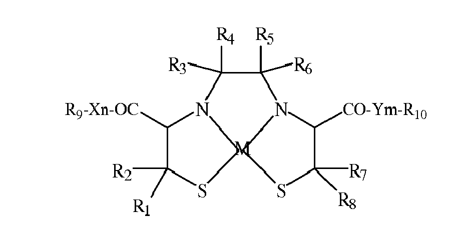 wherein
   R₁, R₂, R₃, R₄ , R₅, R₆, R₇ and R₈ are independently H or CH₃;
   R₉ is H, CH₃, a disease cell cycle targeting compound, a tumor angiogenesis targeting ligand, a tumor apoptosis targeting ligand, a disease receptor targeting ligand, amifostine, angiostatin, monoclonal antibody C225, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine;
   R₁₀ is H, CH₃, disease cell cycle targeting compound, a tumor angiogenesis targeting ligand, a tumor apoptosis targeting ligand, a disease receptor targeting ligand, amifostine, angiostatin, monoclonal antibody C225, monoclonal antibody CD31, monoclonal antibody CD40, capecitabine, COX-2, deoxycytidine, fullerene, herceptin, human serum albumin, lactose, leuteinizing hormone, pyridoxal, quinazoline, thalidomide, transferrin, or trimethyl lysine;
   n is 0 or 1; m is 0 or 1;
   X is a water soluble peptide, C₁-C₂₀ alkyl, glutamic acid, polyglutamic acid, aspartic acid, polyaspartic acid, bromoethylacetate, ethylenediamine or lysine when n is 1, or a bond when n is 0;
   Y is a water soluble peptide, C₁-C₂₀ alkyl, glutamic acid, polyglutamic acid, aspartic acid, polyaspartic acid, bromoethylacetate, ethylenediamine or lysine when m is 1, or a bond when m is 0; and
   M is *^{99m}*Tc, ¹⁸⁸Re, ¹⁸⁶Re, ¹⁸³Sm, ¹⁶⁶Ho ⁹⁰Y, ⁸⁹Sr, ⁶⁷Ga, ⁶⁸Ga, ¹¹¹In, ¹⁸³Gd, ⁵⁹Fe, ²²⁵Ac , ²¹²Bi, ²¹¹At, ⁴⁵Ti, ⁶⁰Cu, ⁶¹Cu, ⁶⁷Cu, ⁶⁴Cu or ⁶²Cu.
3. The compound of embodiment 1, wherein the targeting ligand comprises a tumor angiogenesis targeting ligand.
4. The compound of embodiment 3, wherein the targeting ligand comprises COX-2, anti-EGF, herceptin, angiostatin, or thalidomide.
5. The compound of embodiment 1, wherein the targeting ligand is a disease cell cycle targeting ligand.
6. The compound of embodiment 5, wherein the targeting ligand comprises adenosine, penciclovir, FIAU, FIRU, IVFRU, GCV, PCV, FGCV, FPCV, FHPG, FHBG, or guanine.
7. The compound of embodiment 1, wherein the targeting ligand comprises a tumor apoptosis targeting ligand.
8. The compound of embodiment 7, wherein the targeting ligand comprises a TRAIL or caspase-3 targeting ligand.
9. The compound of embodiment 2, wherein the targeting ligand comprises a disease receptor targeting ligand
10. The compound of embodiment 9, wherein the targeting ligand comprises an estrogen, an androgen, luteinizing hormone, transferrin, or a progestin.
11. The compound of embodiment 1, wherein the targeting ligand comprises carnitine or doxorubicin.
12. The compound of embodiment 1, wherein the targeting ligand comprises penciclovir or adenosine.
13. The compound of embodiment 1, wherein the targeting ligand comprises amifostine.
14. The compound of embodiment 1, wherein the targeting ligand comprises anti-EGF receptor.
15. The compound of embodiment 1, wherein the targeting ligand comprises monoclonal antibody CD31.
16. The compound of embodiment 1, wherein the targeting ligand comprises monoclonal antibody CD40.
17. The compound of embodiment 1, wherein the targeting ligand comprises capecitabine.
18. The compound of embodiment 1, wherein the targeting ligand comprises deoxycytidine.
19. The compound of embodiment 1, wherein the targeting ligand comprises fullerene.
20. The compound of embodiment 1, wherein the targeting ligand comprises human serum albumin.
21. The compound of embodiment 1, wherein the targeting ligand comprises lactose.
22. The compound of embodiment 1, wherein the targeting ligand comprises pyridoxal.
23. The compound of embodiment 1, wherein the targeting ligand comprises quinazoline.
24. The compound of embodiment 1, wherein the targeting ligand comprises trimethyl lysine.
25. The compound of embodiment 1, wherein the targeting ligand comprises a disease cell cycle targeting compound.
26. The compound of embodiment 25, wherein the disease cell cycle targeting compound comprises adenosine, penciclovir, FIAU, FIRU, IVFRU, GCV, PCV, FGCV, FHPG, FHBG, or guanine.
27. The compound of embodiment 1, wherein the N₂S₂ chelate is further defined as ethylenedicysteine.
28. The compound of embodiment 1, further comprising a radioactive nuclide.
29. The compound of embodiment 28, wherein the radioactive nuclide comprises ^{99m}Tc, ¹⁸⁸Re, ¹⁸⁶Re, ¹⁸³Sm, ¹⁶⁶Ho,⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu.
30. The compound of embodiment 1, futher comprising a water soluble peptide, C₁-C₂₀ alkyl, glutamic acid, polyglutamic acid, aspartic acid, polyaspartic acid, bromoethylacetate, ethylenediamine or lysine positioned between the targeting ligand and the chelate.
31. A method of synthesizing a radiolabeled N₂S₂ chelate conjugated to targeting ligand comprising the steps:
   a) obtaining a compound in accordance with embodiment 1;
   b) admixing said compound a radionuclide and a reducing agent to obtain a radionuclide labeled derivative, wherein the N₂S₂ chelate forms a chelate with the radionuclide.
32. The method of embodiment 31, wherein said reducing agent is a dithionite ion, a stannous ion or a ferrous ion.
33. The method of embodiment 31, wherein said radionuclide is ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu.
34. A method of imaging a site within a mammalian body comprising the steps:
   a) administering an effective diagnostic amount of a compound in accordance with embodiment 28 to said site; and
   b) detecting a radioactive signal from said compound localized at a site.
35. The method of embodiment 34, wherein said site is a tumor.
36. The method of embodiment 34, wherein said site is an infection.
37. The method of embodiment 34, wherein said site is breast cancer, ovarian cancer, prostate cancer, endometrium, heart cancer, lung cancer, brain cancer, liver cancer, folate (+) cancer, ER (+) cancer, spleen cancer, pancreas cancer, or intestine cancer.
38. A kit for preparing a radiopharmaceutical preparation comprising:
   a) a sealed container including a predetermined quantity of a compound that is a radionuclide-labeled N₂S₂ chelate-targeting ligand conjugate in accordance with embodiment 1; and
   b) a sufficient amount of a reducing agent.
39. The kit of embodiment 38, further comprising a radionuclide.
40. The kit of embodiment 39, wherein the radionuclide is *^{99m}*Tc*,* ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu.
41. The kit of embodiment 37, further comprising an antioxidant.
42. The kit of embodiment 41, wherein the antioxidant is vitamin C, tocopherol, pyridoxine, thiamine, or rutin.
43. The kit of embodiment 42, wherein the antioxidant is vitamin C.
44. The kit of embodiment 38, further comprising a transition chelator.
45. The kit of embodiment 44, wherein the transition chelator is glucoheptonate, gluconate, glucarate, citrate, or tartarate.
46. The kit of embodiment 45, wherein the transition chelator is gluconate or glucarate.
47. The kit of embodiment 38, wherein the reducing agent is tin (II) chloride or triphenylphosphine.
48. A method of assessing the pharmacology of a agent of interest comprising:
   a) preparing an conjugate of the agent to an N₂S₂ chelate;
   b) adding a radioactive nuclide to said conjugated chelate to form a radioactive conjugate;
   c) administering said radioactive conjugate to a subject; and
   d) assessing the pharmacology of the agent.
49. The method of embodiment 48, wherein the agent of interest is a pharmaceutical agent.
50. The method of embodiment 48, wherein the N₂S₂ chelate is ethylenedicysteine.
51. The method of embodiment 48, wherein the subject is a laboratory animal.
52. The method of embodiment 48, wherein the subject is a human.
53. The method of embodiment 48, wherein assessing the pharmacology of the agent comprises assessing the biodistribution of the agent.
54. The method of embodiment 48, wherein assessing the pharmacology of the agent comprises assessing the biostability of the agent.
55. The method of embodiment 48, wherein assessing the pharmacology of the agent comprises assessing the bioelimination of the agent.

### REFERENCES

The following references have been cited in the preceding body of the description:
US Patents 5,268,163; 5,605,672; 5,817,776; 5,955,053; 5,986,074; and 6,284,220
AU Patent 0175210A5;
PCT applications WO 0175125, WO 0180906, WO 0191807, WO 0232291, WO 9116076, and WO 9428940;
Alauddin et al., Nucl. Med. Biol., 23:787-792, 1996.
Alauddin et al., Nucl. Med. Biol., 26:371-376, 1999.
Alauddin et al., J. Nucl. Med., 42(11):1682-1690, 2001.
Alauddin and Conti, Nucl. Med. Biol., 25:175-180, 1998.
Banerjeei et al., Anticancer Res., 20:2641-2645, 2000.
Bertolini et al., Br. J. Haematol., 106:5045-09, 1999.
Blondeau et al., Can. J. Chem., 45:49-52, 1967.
Bocci et al., Cancer Chemother. Pharmacol., 43:205-212, 1999.
Burian et al., Acta Otolaryngol., 119:289-292, 1999.
Canet et al., Magn. Reson. Med., 43(3):403-409, 2000.
Cao, Haematologica, 84:643-650, 1999.
Dhanabal et al., J. Biol. Chem., 274:11721-11726, 1999.
Duda et al., Cancer Res., 60:1111-1116, 2000.
Frisch and Screaton, Curr. Opin. Cell Biol. 13:555-562, 2001.
Gambhir et al., J. Nucl. Med., 39:2003-2011, 1998.
Gambhir et al., Proc. Natl. Acad. Sci. USA, 96:2333-2338, 1999.
Gambhir et al., Proc. Natl. Acad. Sci. USA, 97:2785-2790, 2000.
Gasparini, Drugs, 58:17-38, 1999.
Goethals et al., J. Nucl. Med., 36:880-882, 1995.
Green and Evan, Cancer Cell, 1:19-30, 2002.
Guang-Wu, et al., Laryngoscope, 110:2066-2069, 2000.
Higashi et al., J. Nucl. Med., 34:414-419, 1992.
Inoue et al., Clin. Cancer Res., 6:4866-4873, 2000.
Iyer et al., J. Nucl. Med., 42:96-105, 2001.
Jiang et al., Am. J. Physiol. Cell Physiol., 280:1140-1150, 2001.
Jouan et al., Blood, 94:984-993, 1999.
Kao et al., Cancer, 83:64-68, 1998.
Laissy et al., Magn. Reson. Imaging, 12(3):413-419, 1994.
Lamszus et al., Neurosurgery, 46:938-948, 2000.
Li et al., Inorg. Chem., 35(2):404-414, 1996.
Liao F et al. , Cancer Res., 60:6805-6810, 2000.
Logothetis et al., Clin. Cancer Res., 7:1198-1203, 2001.
Lozonschi et al., Cancer Res., 59:1252-1258, 1999.
Maekawa et al., Cancer Res., 59:1231-1235, 1999.
Martiat et al., J. Nucl. Med., 29:1633-1637, 1988.
McConathy et al., Nucl. Med. Biol., 30:477-490, 2003.
Minn et al., Cancer, 61:1776-1781, 1988.
Moreira et al., J. Neurooncol., 43:109-114, 1999.
Moulton et al., Circulation, 99:1726-1732, 1999.
Namavari et al., Nucl. Med. Biol., 27:157-162, 2000.
Ohtsuki et al., Eur. J. Nucl. Med., 26(10):1251-1258, 1999.
Okada et al., J. Nucl. Med., 32:686-691, 1991.
Okada et al., J. Nucl. Med., 33:325-329, 1992.
Pedram et al., Endocrinology, 142:1578-1586, 2001.
Petzelbauer et al., Cytokine, 7:267-272, 1995.
Ratner and Clarke, J. AM Chem. Soc., 59:200-206, 1937.
Reed, Cancer Cell, 3:17-22, 2003.
Sakamoto et al., Invest. Ophthalmol. Vis. Sci., 36:1076-1083, 1995.
Shields et al., J. Nucl. Med., 31:337-342, 1990.
Sion-Vardy et al., Pathol. Res. Pract., 197:1-5, 2001.
Slaton et al., Am. J. Pathol., 158:735-743, 2001.
Smith et al., J. Clin. Oncol., 18:2046-2052, 2000.
Smith et al., Ann. Oncol., 10:707-713, 1999.
Taggart et al., Human Mutation., 13(3):210-220, 1999.
Tjuvajev et al., J. Nucl. Med., 43:1072-1083, 2002.
Tschopp et al., Curr. Biol. 9:R381-R384, 1999.
Ugurel et al., J. Clin. Oncol., 19:577-583, 2001.
Van Nerom et al., Eur. J. Nucl. Med., 20:738-746, 1993.
Vriens et al., J. Thorac. Cardiovasc. Surg., 116:844-853, 1998.
Wu et al., J. Diabetes Complications, 17:297-300, 2003.
Xiangming et al., Ann. Surg. Oncol., 5:585-589, 1998.
Yaghoubi et al., J. Nucl. Med., 42:1225-1234, 2001.
Yeh et al., Mol. Pharmacol., 59:1333-1342, 2001.

## Claims

1. A radionuclide-labeled ethylenedicysteine (EC) derivative comprising a disease cell cycle targeting compound, wherein
(i) the EC forms an N₂S₂ chelate with the radionuclide,
(ii) the disease cell cycle targeting compound is a nucleoside or analog thereof and is selected from guanine, adenosine, penciclovir, FIAU, FIRU, FMAU, IVFRU, GCV, PCV, FGCV, FPCV, FHPG, and FHBG, and
(iii)the disease cell cycle targeting compound is conjugated to EC directly or via a linker.

2. The EC derivative of claim 1, further defined as: 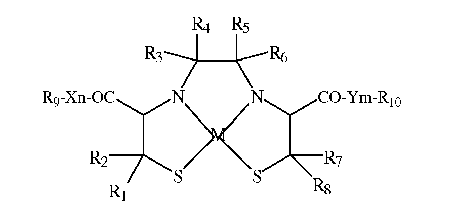 wherein
R₁, R₂, R₃, R₄ , R₅, R₆, R₇ and R₈ are independently H or CH₃;
R₉ is H, CH₃, or a disease cell cycle targeting compound as defined in claim 1;
R₁₀ is H, CH₃, or a disease cell cycle targeting compound as defined in claim 1; n is 0 or 1;
m is 0 or 1;
X is a water soluble peptide, C₁-C₂₀ alkylene, glutamic acid, polyglutamic acid, aspartic acid, polyaspartic acid, bromoethylacetate, ethylenediamine, or lysine, when n is 1, or a bond when n is 0;
Y is a water-soluble peptide, C₁-C₂₀ alkylene, glutamic acid, polyglutamic acid, aspartic acid, polyaspartic acid, bromoethylacetate, ethylenediamine or lysine, when m is 1, or a bond when m is 0; and
M is ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu, or ⁶²Cu.

3. A method of synthesizing the radionuclide-labeled ethylenedicysteine (EC) derivative of claim 1, the method comprising the steps of:
a) obtaining an ethylenedicysteine (EC) derivative comprising a disease cell cycle targeting compound, wherein
(i) the EC is capable of forming an N₂S₂ chelate with a radionuclide,
(ii) the disease cell cycle targeting compound is as defined in claim 1, and
(iii)the disease cell cycle targeting compound is conjugated to EC directly or via a
linker;
b) admixing said derivative to a radionuclide and a reducing agent to obtain the radionuclide-labeled EC derivative, wherein the EC derivative forms a chelate with the radionuclide.

4. The method of claim 3, wherein said reducing agent is a dithionite ion, a stannous ion, or a ferrous ion.

5. The EC derivative of claim 1 or 2 for use in a method of imaging a site within a mammalian body.

6. The EC derivative for the use of claim 5, wherein said site is a tumor.

7. The EC derivative for the use of claim 6, wherein said site is breast, ovarian, prostate, endometrium, heart, lung, brain, spleen, pancreas, intestine, or liver cancer, or a folate (+) or ER (+) cancer.

8. A kit for preparing a radiopharmaceutical preparation, the kit comprising:
a) a sealed container including a predetermined quantity of the ethylenedicysteine (EC) derivative as defined in claim 1; and
b) a sufficient amount of a reducing agent.

9. The kit of claim 8, the kit further comprising a radionuclide.

10. The kit of claim 9, wherein the radionuclide is *^{99m}*Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu ⁶⁷Cu, ⁶⁴Cu or ⁶²Cu.

11. The kit of claim 8, the kit further comprising an antioxidant.

12. The kit of claim 11, wherein the antioxidant is vitamin C, tocopherol, pyridoxine, thiamine, or rutin.

13. The kit of claim 8, the kit further comprising a transition chelator.

14. The kit of claim 13, wherein the transition chelator is glucoheptonate, gluconate, glucarate, citrate, or tartarate.

15. The kit of claim 8, wherein the reducing agent is tin (II) chloride or triphenylphosphine.
